# EUROPEAN PATENT SPECIFICATION

(11) **EP 3 125 959 B1**
(45) Date of publication and mention of the grant of the patent: **07.04.2021**
(21) Application number: 15774154.7
(22) Date of filing: 20.02.2015
(51) Int. Cl.: A61L 17/08

(54) **ELECTROSPUN BIOCOMPATIBLE FIBER COMPOSITIONS**
ELEKTROGESPONNENE BIOKOMPATIBLE FASERZUSAMMENSETZUNGEN
COMPOSITIONS DE FIBRES BIOCOMPATIBLES ÉLECTROFILÉES

(30) Priority: 04.04.2014 US 201461975586 P
(43) Date of publication of application: 08.02.2017
(73) Proprietor: Nanofiber Solutions, LLC, Wilmington, DE 19801 (US)
(72) Inventor: JOHNSON, Jed, London, Ohio 43140 (US); KAYUHA, Ross, Dublin, Ohio 43017 (US)
(74) Representative: HGF
(86) International application number: PCT/US2015/016973
(87) International publication number: WO 2015/153011

(56) References cited:
- WO-A1-2014/145864
- US-A1- 2002 090 725
- US-A1- 2005 277 985
- US-A1- 2010 166 854
- US-A1- 2010 303 881
- US-A1- 2011 028 834
- US-A1- 2013 103 079
- HE ET AL.: 'Fabrication of drug-loaded electrospun aligned fibrous threads for suture applications.' JOURNAL OF BIOMEDICAL MATERIALS RESEARCH PART A. vol. 89, no. 1, 2009, pages 80 - 95, XP055229026
- N.V.HERRERA, A.P.MATHEW, L.Y.WA AND K.OKSMAN: "Randomly oriented and aligned cellulose fibres reinforced with cellulose nanowhiskers prepared by electrospinning", PLASTIC, RUBBER AND COMPOSITES, vol. 20, no. 2, 12 November 2013 (2013-11-12), - 12 November 2013 (2013-11-12), pages 57-64,
- J LEE AND Y DENG: "Increased Mechanical Properties of Aligned and Isotropic Electrospun PVA Nanofiber Webs by Cellulose Nanowhisker Reinforcement", MOLECULAR RESEARCH, vol. 20, no. 1, 2012, pages 76-83,

## Description

### CLAIM OF PRIORITY

This application claims priority to and benefit of U.S. Provisional Application Serial No. 61/975,586 filed April 4, 2014, entitled "Electrospun Biocompatible Fiber Compositions,".

### SUMMARY

In an embodiment, there is provided a kit comprising:
a first component comprising;
a plurality of micronized electrospun fiber fragments comprising a polymer, wherein the plurality of electrospun fiber fragments have an average length of 0.9 µm to 1100 µm: and an average diameter of 0.09 µm to 11 µm;
a plurality of electrospun fiber fragment clusters consisting of the polymer, wherein the plurality of electrospun fiber fragment clusters have, independently, an average length of 0.9 µm to 1100 µm, an average width of 0.9 µm to 1100 µm, and an average height of 0.9 µm to 1100 µm; and
a second component comprising a carrier medium.

In an embodiment, a composition comprises
a plurality of micronized electrospun fiber fragments comprising a polymer, wherein the plurality of electrospun fiber fragments have an average length of 0.9 µm to 1100 µm; and an average diameter of 0.09 µm to 11 µm;
a plurality of electrospun fiber fragment clusters consisting of the polymer, wherein the plurality of electrospun fiber fragment clusters have independently, an average length of 0.9 µm to 1100 µm, an average width of 0.9 µm to 1100 µm, and an average height of 0.9 µm to 1100 µm; and, and
a carrier medium.

Described herein is a therapeutic composition which may include a plurality of electrospun fiber fragments comprising at least one polymer, having an average length of about 10 µm to about 1000 µm, and an average diameter of about 0.1 µm to about 10 µm, a carrier medium, and a plurality of cells.

Described herein is a micronized biocompatible textile which may include a plurality of micronized electrospun fiber fragments comprising at least one polymer, having an average length of about 10 µm to about 1000 µm, and an average diameter of about 0.1 µm to about 10 µm.

Described herein is a biocompatible suture which may include at least one electrospun fiber comprising at least one polymer, in which the suture has a metric gauge of about 0.01 to about 3.

Described herein is a method of making a biocompatible suture which may include electrospinning a polymer solution onto a receiving surface, thereby forming at least one non-overlapping nanofiber thread, removing the at least one nanofiber thread from the receiving surface, and cutting the at least one nanofiber thread into one or more biocompatible sutures having a length of about 1 cm to about 50 cm.

### BRIEF DESCRIPTION OF THE DRAWINGS

FIG. 1 illustrates an embodiment of a system for electrospinning a polymer fiber onto a mandrel in accordance with the present disclosure.
FIG. 2A illustrates an embodiment of a system for forming electrospun fibers as windings on a mandrel surface in accordance with the present disclosure.
FIG. 2B illustrates an embodiment of a system for forming electrospun fibers as threads along the longitudinal axis of a mandrel in accordance with the present disclosure.
FIGS. 3A and 3B depict a biocompatible textile formed without the addition of a water-soluble material and a biocompatible textile formed with the addition of a water-soluble material, respectively, in accordance with the present description.
FIG. 4 depicts an electrospun fiber suture in accordance with the present description.
FIGS. 5A and 5B depict low-magnification and high-magnification images, respectively, of a micronized electrospun textile in accordance with the present description.
FIGS. 6A, 6B, 6C, and 6D depict scanning electron microscope images of a micronized electrospun textile when exposed to adipose-derived stem cells, at 0 minutes after exposure to stem cells, at 5 minutes after exposure to stem cells, at 25 minutes after exposure to stem cells, and at 30 minutes after exposure to stem cells, respectively, in accordance with the present description.

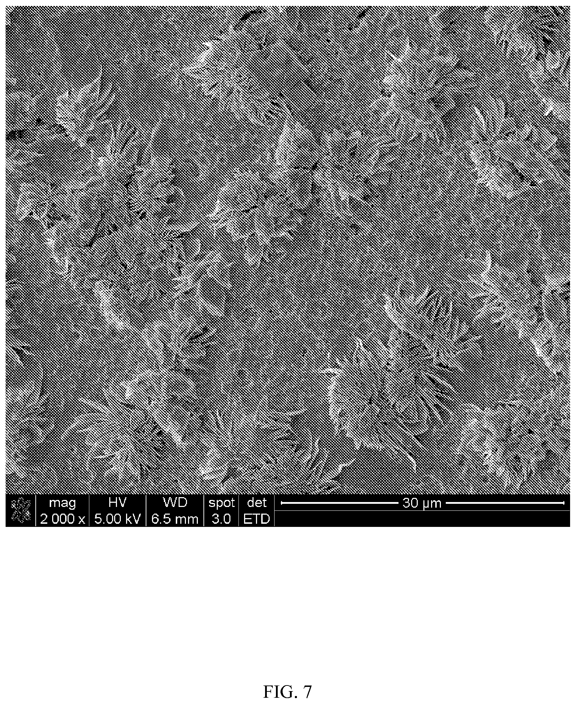

FIG. 7 depicts a scanning electron microscope image of platelet-rich plasma combined with a micronized electrospun textile at 0 minutes after exposure. "Nanowhiskers" are barely visible, due to the rapid attachment of platelets to the fibers.

### DETAILED DESCRIPTION

In order to repair or replace organs in patients, two independent and often mutually exclusive parameters must be met. First, any implant must have structural integrity, including a certain amount of rigidity to remain in place once implanted. Second, an implant must be biocompatible so that it is not rejected by the body, and so that it does not create damaging inflammation. In certain instances, the infusion, attachment, adhesion, penetration, and proliferation of cells is also required for an implant to function as an organ or biological component, as opposed to a simple prosthesis. No prior implant has been able to embody such parameters. For example, decellularized organ scaffolds, gels, and polymer matrices have been implanted, but one or more mechanical or biological issues have arisen. As such, none of these systems have been able to provide the appropriate mechanical properties, along with the necessary biocompatibility.

This disclosure is not limited to the particular systems, devices and methods described as these may vary within the scope of the appended claims. The terminology used in the description is for the purpose of describing the particular versions or embodiments only, and is not intended to limit the scope of the disclosure.

The following terms shall have, for the purposes of this application, the respective meanings set forth below. Unless otherwise defined, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention.

As used herein and in the appended claims, the singular forms "a," "an," and "the" include plural references, unless the context clearly dictates otherwise. Thus, for example, reference to a "fiber" is a reference to one or more fibers and equivalents thereof known to those skilled in the art, and so forth.

As used herein, the term "about" means plus or minus 10% of the numerical value of the number with which it is being used. Therefore, about 50% means in the range of 45%-55%.

"Optional" or "optionally" means that the subsequently described event or circumstance may or may not occur, and that the description includes instances where the event occurs and instances where it does not.

As used herein, the term "therapeutic" means an agent utilized to treat, combat, ameliorate, prevent or improve an unwanted condition or disease of a patient. In part, embodiments of the present disclosure are directed to the treatment of wounds, injuries of tendons, ligaments, or other musculoskeletal structures, organs, and the like.

When used in conjunction with a therapeutic, "administering" means to administer a therapeutic directly into or onto a target tissue, or to administer a therapeutic to a patient whereby the therapeutic positively impacts the tissue to which it is targeted. The compositions of the present disclosure can be administered in the conventional manner by any method in which they are effective. "Administering" may be accomplished by parenteral, intravenous, intramuscular, subcutaneous, intraperitoneal, or any other injection, oral or topical administration, suppository administration, inhalation, or by such methods in combination with other known techniques.

In some embodiments, the compounds, compositions, and methods disclosed herein can be utilized with or on a subject in need of treatment, which can also be referred to as "in need thereof." As used herein, the phrase "in need thereof' means that the subject has been identified as having a need for the particular method or treatment and that the treatment has been given to the subject for that particular purpose.

The term "subject" as used herein includes, but is not limited to, humans, non-human vertebrates, and animals such as wild, domestic, and farm animals. Preferably, the term "subject" refers to mammals. More preferably, the term "subject" refers to humans.

A "therapeutically effective amount" or "effective amount" of a composition is a predetermined amount calculated to achieve the desired effect, i.e., to improve, localize, increase, inhibit, block, or reverse the adhesion, activation, migration, penetration, or proliferation of cells. The activity contemplated by the present methods includes medical, therapeutic, cosmetic, aesthetic, and/or prophylactic treatment, as appropriate. The specific dose of a compound administered according to this disclosure to obtain therapeutic, cosmetic, aesthetic, and/or prophylactic effects will, of course, be determined by the particular circumstances surrounding the case, including, for example, the compound administered, the route of administration, and the condition being treated. The compounds are effective over a wide dosage range. It will be understood that the effective amount administered will be determined by the physician, veterinarian, or other medical professional in the light of the relevant circumstances including the condition to be treated, the choice of compound to be administered, and the chosen route of administration, and therefore the dosage ranges described herein are not intended to limit the scope of the disclosure in any way.

The terms "treat," "treated," or "treating" as used herein refer to both therapeutic treatment and prophylactic or preventative measures, wherein the object is to prevent or slow down (lessen) or entirely reverse (eradicate) an undesired physiological condition, disorder or disease, or to obtain beneficial or desired clinical results. For the purposes of this disclosure, beneficial or desired clinical results include, but are not limited to, alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; remission (whether partial or total), whether detectable or undetectable, or enhancement or improvement of the condition, disorder, or disease; and eradication of the condition, disorder, or disease. Treatment includes eliciting a clinically significant response without excessive levels of side effects. Treatment also includes prolonging survival as compared to expected survival if not receiving treatment.

A textile may have a luminal structure composed of multiple windings of one or more biocompatible fibers. The term "textile" is defined herein as a spun, woven, or otherwise fabricated material comprising fibers. The fibers may be wound about a mandrel, as threads are wound around a bobbin. The fibers may be deposited, in an essentially parallel manner, along a linear dimension of a mandrel or other surface form. Winding the textile may use electrospinning techniques.

"Pore size" is thus defined herein as being the diameter of introduced pores, pockets, voids, holes, spaces, etc. introduced in an unmeshed structure such as a block polymer, polymer sheet, or formed polymer scaffold, and is specifically distinguished from "mesh size" as disclosed herein.

As used herein, the term "mesh size" is the number of openings in a textile per linear measure. For example, if the textile has 1200 openings per linear millimeter, the textile is defined 1200 mesh (e.g., sufficient to allow a 12 micron red blood cell to pass), which is easily convertible between imperial and metric units. A mesh size may be determined based on the number of fibers having a specified average diameter and an average opening size between adjacent fibers along a specified linear dimension. Thus, a textile composed of 10 µm average diameter fibers having 10 µm average diameter openings between adjacent fibers may have about 50 total openings along a 1 mm length and may therefore be defined as a 50 mesh textile.

As used herein, the term "fragment" refers to a portion of a particular fiber. In some embodiments, a fragment may comprise at least one polymer, having an average length of about 10µm to about 1000µm, and an average diameter of about 0.1µm to about 10µm. In some embodiments, a composition may contain a plurality of fragments, a carrier medium, and, optionally, a plurality of cells. Some non-limiting examples of average fragment lengths may include an average length of about 10µm, an average length of about 20µm, an average length of about 30µm, an average length of about 40µm, an average length of about 50µm, an average length of about 75µm, an average length of about 90µm, an average length of about 95µm, an average length of about 100µm, an average length of about 105µm, an average length of about 110µm, an average length of about 150µm, an average length of about 200µm, an average length of about 300µm, an average length of about 400µm, an average length of about 500µm, an average length of about 600µm, an average length of about 700µm, an average length of about 800µm, an average length of about 900µm, an average length of about 1000µm, or ranges between any two of these values (including endpoints). Some non-limiting examples of average fragment diameters may include an average diameter of about 0.1µm, an average diameter of about 0.5µm, an average diameter of about 1µm, an average diameter of about 2µm, an average diameter of about 3µm, an average diameter of about 4µm, an average diameter of about 5µm, an average diameter of about 6µm, an average diameter of about 7µm, an average diameter of about 8µm, an average diameter of about 9µm, an average diameter of about 10µm, or ranges between any two of these values (including endpoints). When combined with a carrier medium, the resulting mixture may include from about 1 fragment per mm³ to about 100,000 fragments per mm³. Some non-limiting examples of mixture densities may include about 2 fragments per mm³, about 100 fragments per mm³, about 1,000 fragments per mm³, about 2,000 fragments per mm³, about 5,000 fragments per mm³, about 10,000 fragments per mm³, about 20,000 fragments per mm³, about 30,000 fragments per mm³, about 40,000 fragments per mm³, about 50,000 fragments per mm³, about 60,000 fragments per mm³, about 70,000 fragments per mm³, about 80,000 fragments per mm³, about 90,000 fragments per mm³, about 100,000 fragments per mm³, or ranges between any two of these values (including endpoints).

As used herein, the term "cluster" refers to an aggregate of fiber fragments, or a linear or curved three-dimensional group of fiber fragments. In some embodiments, a cluster may comprise at least one polymer. Clusters may have a range of shapes. Non-limiting examples of cluster shapes may include spherical, globular, ellipsoidal, and flattened cylinder shapes. Clusters have, independently, an average length of about 1 µm to about 1000 µm, an average width of about 1 µm to about 1000 µm, and an average height of about 1 µm to about 1000 µm. It may be appreciated that any cluster dimension, such as length, width, or height, is independent of any other cluster dimension. Some non-limiting examples of average cluster dimensions include an average dimension (length, width, height, or other measurement) of about 1µm, an average dimension of about 5µm, an average dimension of about 10µm, an average dimension of about 20µm, an average dimension of about 30µm, an average dimension of about 40µm, an average dimension of about 50µm, an average dimension of about 75µm, an average dimension of about 90µm, an average dimension of about 95µm, an average dimension of about 100µm, an average dimension of about 105µm, an average dimension of about 110µm, an average dimension of about 150µm, an average dimension of about 200µm, an average dimension of about 300µm, an average dimension of about 400µm, an average dimension of about 500µm, an average dimension of about 600µm, an average dimension of about 700µm, an average dimension of about 800µm, an average dimension of about 900µm, an average dimension of about 1000µm, or ranges between any two of these values (including endpoints), or independent combinations of any of these ranges of dimensions. Clusters may include an average number of about 2 to about 1000 fiber fragments. Some non-limiting examples of average numbers of fiber fragments per cluster include an average of about 2 fiber fragments per cluster, an average of about 5 fiber fragments per cluster, an average of about 10 fiber fragments per cluster, an average of about 20 fiber fragments per cluster, an average of about 30 fiber fragments per cluster, an average of about 40 fiber fragments per cluster, an average of about 50 fiber fragments per cluster, an average of about 60 fiber fragments per cluster, an average of about 70 fiber fragments per cluster, an average of about 80 fiber fragments per cluster, an average of about 90 fiber fragments per cluster, an average of about 100 fiber fragments per cluster, an average of about 110 fiber fragments per cluster, an average of about 200 fiber fragments per cluster, an average of about 300 fiber fragments per cluster, an average of about 400 fiber fragments per cluster, an average of about 500 fiber fragments per cluster, an average of about 600 fiber fragments per cluster, an average of about 700 fiber fragments per cluster, an average of about 800 fiber fragments per cluster, an average of about 900 fiber fragments per cluster, an average of about 1000 fiber fragments per cluster, or ranges between any two of these values (including endpoints). In some embodiments, a composition may contain a plurality of fragments, a plurality of clusters, a carrier medium, and, optionally, a plurality of cells.

As used herein, the term "implant" may refer to any structure that may be introduced for a permanent or semi-permanent period of time into a body. An implant may have the shape and size of a native organ or tissue that it may serve to replace. Alternatively, an implant may have a shape not related to a specific bodily organ or tissue. In yet another embodiment, an implant may have a shape of an entire bodily organ or tissue, or only a portion thereof. In still another example, an implant may be shaped like a portion of a bodily organ or tissue, or may simply comprise a patch of material. In still another example, a composition for implantation may be flexible and not rigidly shaped, and may mold or form to the area to which it is applied. The implant may be designed for introduction into a body of an animal, including a human.

The compositions provided herein may be for use in biocompatible implants for patients. In certain embodiments, a polymer fiber is used such as polyurethane and/or polyethylene terephthalate. In some embodiments, a polymer fiber is spun over a mandrel so as to form a textile roll or tube. In some embodiments, the thickness of the textile roll or tube may be regulated by changing the number of rotations of the mandrel over time while the textile roll or tube collects the fiber. In certain other embodiments, the biocompatible textile has a mesh size of about 1 opening per mm to about 20 openings per mm. Some non-limiting examples of mesh sizes may include about 1 opening per mm, about 2 openings per mm, about 4 openings per mm, about 6 openings per mm, about 8 openings per mm, about 10 openings per mm, about 15 openings per mm, about 20 openings per mm, or ranges between any two of these values (including endpoints). In some embodiments, the mesh size of the spun textile may be about 20 openings per mm to about 500 openings per mm. Some non-limiting examples of mesh sizes may include about 20 openings per mm, about 40 openings per mm, about 60 openings per mm, about 80 openings per mm, about 100 openings per mm, about 200 openings per mm, about 300 openings per mm, about 400 openings per mm, about 500 openings per mm, or ranges between any two of these values (including endpoints). In other embodiments, the mesh size may be about 500 openings per mm to about 1000 openings per mm. Some non-limiting examples of mesh sizes may include about 500 openings per mm, about 600 openings per mm, about 700 openings per mm, about 800 openings per mm, about 1000 openings per mm, or ranges between any two of these values (including endpoints). In some embodiments, the mesh size of the textile may be regulated by changing the speed and direction by which the fiber is deposited onto the mandrel, such as, by example, moving the position and direction in which the thread is spun onto the textile roll or tube.

Described herein is the use of a textile as described herein as a biocompatible implant. The textile having a mesh size may not only provide lightweight and flexible mechanical support, but also may allow cells to migrate into and throughout the mesh over time and may improve the biocompatibility of the implant. The textile may provide sufficient structural rigidity so as to be surgically secured into an implant site while retaining sufficient flexibility under load stresses such as compression, shear, and torsion so as to allow the patient to physically move about once the implant is in place.

The textile may include an additional surface treatment of the polymer fiber which can be used to modulate and enhance cellular attachment to the fibers. The textile may not cause untreatable inflammation or rejection when implanted in a patient. As such, the textile implant may not be subject to rejection or life-threating inflammation within 1 day, 3 days, 5 days, 7 days, 2 weeks, 3 weeks, a month or longer after implantation. The textile implant can be retained in the patient for at least 1 day, 3 days, 5 days, 7 days, 2 weeks, 3 weeks, a month or longer. The implant may be retained in the patient for 6 months, a year, a term of years, or the lifetime of the patient. In some of the disclosed examples, spaces may be introduced through directional application of a polymer thread to a mandrel or other surface form during the electrospinning process as described herein. Spaces may be introduced through the addition of particulates to the textile as the polymer fiber is deposited on a mandrel or other surface form during the electrospinning process as described herein.

While it is contemplated that any method or composition consistent with the disclosure is embodied, electrospun textiles may have specific advantages that are useful for implants. For example, when an electrospun textile is formed from a polymer network deposed on a mandrel, the traditional molding processed can be bypassed since the polymer already exists in the form of a thread before the implant is shaped. As such, a textile-based approach to creating biocompatible implants allows control of four critical parameters that cannot be controlled using inherently combined polymerization and molding processes. First, since the polymer is formed as a thread as part of the electrospinning process, there is no need for setting, curing or other time-consuming processes. Second, the polymer fiber itself can be directed to any orientation for mesh spacing without resorting to chemical processes. The formation of a mesh obviates the need to create pores in an otherwise solid form. Third, the mesh size itself can be adjusted to be larger or smaller to promote ingrowth and proliferation of cells. Where such meshes are used to provide structural support for growing and migrating cells, the mesh may also operate as a cellular scaffold, in addition to conferring the other advantages disclosed herein. A particle size can similarly be identified by the size of the mesh opening such as with the US sieve size, Tyler equivalent, mm, or inches. Fourth, the mesh can be sized to provide structural integrity such as rebound from deformation, flexibility under load, and other advantageous mechanical properties.

### Spinning textiles

The biocompatible textiles disclosed herein may be manufactured by any method. One non-limiting method may include break or open-end spinning, in which slivers are blown by air onto a rotating drum where they attach themselves to the tail of a formed textile (such as thread, rope or yarn) that is continually being drawn out from the drum. Other non-limiting methods may include ring spinning and mule spinning. In certain embodiments, a spinning machine may take a roving, thin it and twist it, thereby creating a yarn which may be wound onto a bobbin.

In mule spinning, the roving is pulled off a bobbin and fed through rollers, which feed at several different speeds, thinning the roving at a consistent rate. The thread, rope or yarn is twisted through the spinning of the bobbin as the carriage moves out, and is rolled onto a cop as the carriage returns. Mule spinning produces a finer thread than ring spinning. The mule process is an intermittent process, because the frame advances and returns a specific distance, which can produce a softer, less twisted thread favored for fines and for weft. The ring process is a continuous process, the yarn being coarser, and having a greater twist thereby being stronger and better suited to be warped. Similar methods have various improvements such as a flyer and bobbin and cap spinning.

### Electrospinning textiles

Electrospinning is a method of spinning a polymer fiber or polymer nanofiber from a polymer solution by applying a high DC voltage potential between the polymer solution (or polymer injection system containing the polymer solution) and a receiving surface for the electrospun polymer nanofibers. The voltage potential may include voltages less than or equal to about 15 kV. The polymer may be ejected by a polymer injection system at a flow rate of less than or equal to about 5 mL/h. As the polymer solution travels from the polymer injection system toward the receiving surface, it may be elongated into sub-micron diameter electrospun polymer nanofibers, typically in the range of about 0.1 µm to about 10 µm. Some non-limiting examples of electrospun polymer nanofiber diameters may include about 0.1 µm, about 0.2 µm, about 0.5 µm, about 1µm, about 2µm, about 5 µm, about 10µm, about 20µm, or ranges between any two of these values (including endpoints).

A polymer injection system may include any system configured to eject some amount of a polymer solution into an atmosphere to permit a flow of the polymer solution from the injection system to the receiving surface. In some non-limiting examples, the injection system may deliver a continuous stream of a polymer solution to be formed into a polymer nanofiber. In alternative examples, the injection system may be configured to deliver intermittent streams of a polymer to be formed into multiple polymer nanofibers. In one embodiment, the injection system may include a syringe under manual or automated control. In another embodiment, the injection system may include multiple syringes under individual or combined manual or automated control. In some examples, a multi-syringe injection system may include multiple syringes, each syringe containing the same polymer solution. In alternative examples, a multi-syringe injection system may include multiple syringes, each syringe containing a different polymer solution.

The receiving surface may move with respect to the polymer injection system, or the polymer injection system may move with respect to the receiving surface. In some embodiments, the receiving surface may move with respect to the polymer injection system under manual control. In other embodiments, the surface may move with respect to the polymer system under automated control. In such embodiments, the receiving surface may be in contact with or mounted upon a support structure that may be moved using one or more motors or motion control systems. In some non-limiting examples, the surface may be a roughly cylindrical surface configured to rotate about a long axis of the surface. In some other non-limiting examples, the surface may be a flat surface that rotates about an axis approximately coaxial with the polymer fiber ejected by the polymer injection system. In yet some other non-limiting examples, the surface may be translated in one or more of a vertical direction and a horizontal direction with respect to the polymer nanofiber ejected by the polymer injection system. It may be further recognized that the receiving surface of the polymer nanofiber may move in any one direction or combination of directions with respect to the polymer nanofiber ejected by the polymer injection system. The pattern of the electrospun polymer nanofiber deposited on the receiving surface may depend upon the one or more motions of the receiving surface with respect to the polymer injection system. In one non-limiting example, a roughly cylindrical receiving surface, having a rotation rate about its long axis that is faster than a translation rate along a linear axis, may result in a roughly helical deposition of an electrospun polymer fiber forming windings about the receiving surface. In an alternative example, a receiving surface having a translation rate along a linear axis that is faster than a rotation rate about a rotational axis, may result in a roughly linear deposition of an electrospun polymer fiber along a liner extent of the receiving surface.

In some embodiments, the receiving surface may be coated with a non-stick material, such as, for example, aluminum foil, a stainless steel coating, polytetrafluoroethylene, or a combination thereof, before the application of the electrospun polymer nanofibers. The receiving surface, such as a mandrel, may be fabricated from aluminum, stainless steel, polytetrafluoroethylene, or a combination thereof to provide a non-stick surface on which the electrospun nanofibers may be deposited. In other embodiments, the receiving surface may be coated with a simulated cartilage or other supportive tissue. In some non-limiting examples, the receiving surface may be composed of a planar surface, a circular surface, an irregular surface, and a roughly cylindrical surface. One embodiment of a roughly cylindrical surface may be a mandrel. A mandrel may take the form of a simple cylinder, or may have more complex geometries. In some non-limiting examples, the mandrel may take the form of a hollow bodily tissue or organ. In some non-limiting examples, the mandrel may be matched to a subject's specific anatomy. Non-limiting embodiments of such bodily tissues may include a trachea, one or more bronchi, an esophagus, an intestine, a bowel, a ureter, a urethra, a blood vessel, or a nerve sheath (including the epineurium or perineurium).

The polymer solution may be a fluid composed of a polymer liquid by the application of heat. Alternatively, the polymer solution can comprise any polymer or combination of polymers dissolved in a solvent or combination of solvents. The concentration range of polymer or polymers in solvent or solvents may be, without limitation, about 1 wt% to about 50 wt%. Some non-limiting examples of polymer concentration in solution may include about 1 wt%, 3 wt%, 5 wt%, about 10 wt%, about 15 wt%, about 20 wt%, about 25 wt%, about 30 wt%, about 35 wt%, about 40 wt%, about 45 wt%, about 50 wt%, or ranges between any two of these values (including endpoints).

### Polymers

In accordance with embodiments herein, a polymer solution used for electrospinning may typically include synthetic or semi-synthetic polymers such as, without limitation, a polyethylene terephthalate, a polyester, a polymethylmethacrylate, polyacrylonitrile, a silicone, a polyurethane, a polycarbonate, a polyether ketone ketone, a polyether ether ketone, a polyether imide, a polyamide, a polystyrene, a polyether sulfone, a polysulfone, a polycaprolactone (PCL), a polylactic acid (PLA), a polyglycolic acid (PGA), a polyglycerol sebacic, a polydiol citrate, a polyhydroxy butyrate, a polyether amide, a polydiaxanone, and combinations or derivatives thereof. Alternative polymer solutions used for electrospinning may include natural polymers such as fibronectin, collagen, gelatin, hyaluronic acid, chitosan, or combinations thereof. It may be understood that electrospinning solutions may also include a combination of synthetic polymers and naturally occurring polymers in any combination or compositional ratio. In some non-limiting examples, the polymer solution may comprise a weight percent ratio of polyethylene terephthalate to polyurethane of about 10% to about 90%. Non-limiting examples of such weight percent ratios may include 10%, 25%, 33%, 50%, 66%, 75%, 90%, or ranges between any two of these values.

The type of polymer in the polymer solution may determine the characteristics of the biocompatible textile, fiber, fragment, or cluster. Some textiles, fibers, fragments, or clusters may be composed of polymers that are bio-stable and not absorbable or biodegradable when implanted. Such textiles, fibers, fragments, or clusters may remain generally chemically unchanged for the length of time in which they remain implanted. Alternative textiles, fibers, fragments, or clusters may be composed of polymers that may be absorbed or bio-degraded over time. Such textiles, fibers, fragments, or clusters may act as an initial template for the repair or replacement of organs and/or tissues. These organ or tissue templates may degrade *in vivo* once the tissue or organs have been replaced or repaired by natural structures and cells. It may be further understood that a biocompatible textile, fiber, fragment, or cluster may be composed of more than one type of polymer, and that each polymer therein may have a specific characteristic, such as stability or biodegradability.

Polymer solutions may also include one or more solvents such as acetone, dimethylformamide, dimethylsulfoxide, N-methylpyrrolidone, acetonitrile, hexanes, ether, dioxane, ethyl acetate, pyridine, toluene, xylene, tetrahydrofuran, trifluoroacetic acid, hexafluoroisopropanol, acetic acid, dimethylacetamide, chloroform, dichloromethane, water, alcohols, ionic compounds, or combinations thereof.

The polymer solutions may also include additional materials. Non-limiting examples of such additional materials may include radiation opaque materials, electrically conductive materials, fluorescent materials, luminescent materials, antibiotics, growth factors, vitamins, cytokines, steroids, anti-inflammatory drugs, small molecules, sugars, salts, peptides, proteins, cell factors, DNA, RNA, or any other materials to aid in non-invasive imaging, or any combination thereof. In some embodiments, the radiation opaque materials may include, for example, barium, tantalum, tungsten, iodine, or gadolinium. In some embodiments, the electrically conductive materials may include, for example, gold, silver, iron, or polyaniline.

### Incorporation of an Anti-Static Bar

During electrospinning, polymer nanofibers are driven toward a receiving surface by charge separation caused by an applied voltage. The receiving surface typically is a conductive surface, composed of, for example, aluminum or copper. The receiving surface may be covered by a thin layer of plastic, ranging, for example, between about 0.001 inches (about 0.025 mm) to about 0.1 inches (about 2.5 mm) thick. The force that drives the electrospun polymer solution from the polymer injection system toward the receiving surface is derived from mobile ions within the polymer solution or melt. The polymer solution ejected by the polymer injection system may have a net positive or negative charge, depending upon the polarity of the voltage applied to the polymer injections system and the receiving surface. When the electrospun polymer nanofiber is deposited on the collector surface to form polymer nanofiber threads, a charge will build up as subsequent nanofiber thread layers are collected. It is believed that as the charge builds up on the receiving surface, the nanofibers threads, having a similar charge, will be repelled. This electrostatic repelling force may, thus, lead to irregularly arranged nanofiber threads that will have a lower degree of alignment. In order to reduce the effects of surface charge on the receiving surface or its polymer nanofiber threads, the use of an anti-static device, such as a bar, may be incorporated into the process to improve nanofiber thread alignment. The anti-static bar bombards the receiving surface with positively or negatively charged ions in the form of, for example, a plasma or corona discharge, thereby neutralizing the charge on the receiving surface. Therefore, as fiber builds up over the receiving surface, successive layers of nanofiber threads will deposit more uniformly in a side-by-side arrangement (parallel relationship) to increase the alignment. The position of an anti-static bar may be parallel to the surface of the receiving surface, (for example, a roughly cylindrical mandrel, wheel, device, or plate) and may be, for example, about 0.5 inches (about 13 mm) to about 3 inches (about 75 mm) away from the receiving surface.

Experimental results demonstrated that nanofiber thread alignment on the receiving surface was improved significantly with the addition of an anti-static bar, when compared to samples spun under the same conditions without the anti-static bar. For example, the alignment of nanofiber threads can be about 83% at a low angle orientation when deposited on a receiving surface lacking an anti-static bar. However, a 12% increase in fiber alignment - to about 95% - may be observed with the use of an anti-static bar. The use of an anti-static bar may also lead to improvements in nanofiber thread alignment on the receiving surface for processes incorporating polymer injection systems having multiple syringes. When nanofiber threads are deposited on a receiving surface lacking an anti-static bar, the nanofiber thread alignment can be low, with about 74% of nanofiber threads deposited in a low angle orientation. With an anti-static bar, under the same spinning conditions, the alignment can become about 92%. It may be appreciated that additional embodiments may include processes that incorporate the use of more than one anti-static bar.

### Combined High Velocity and Alternating Ground Alignment

Enhanced alignment of polymer nanofibers produced during electrospinning has been achieved by various methods including, for example, high velocity fiber collection (for example, on a receiving surface, such as a mandrel, rotating at a high velocity) and fiber collection between sequentially placed electrodes on the rotating receiving surface. The receiving surface may be the rim surface of a metal spoked wheel. The rim surface of the wheel may be coated with a thin insulating layer, such as, for example, a thin layer of polystyrene or polystyrene film. In one non-limiting example, the sequential electrodes may be fabricated from conductive tape placed widthwise across the insulating material. At least one end of each tape electrode may contact one or more metal wheel spokes. The conductive tape may be composed of, for example, carbon or copper and may have a width of about 0.1 inches (0.25 cm) to about 2 inches (about 5 cm). In some embodiments, the conductive tape may be spaced in uniform intervals around the wheel rim. The intervals between the conductive tape and the insulating surface may create alternating layers of conductive and non-conductive surfaces. The metal spokes may be in electrical contact with the source of the electrospinning voltage providing the voltage difference between the polymer injection system and the receiving surface. The combination of a high speed rotational surface and a multiply grounded electrical surface may lead to enhanced fiber alignment.

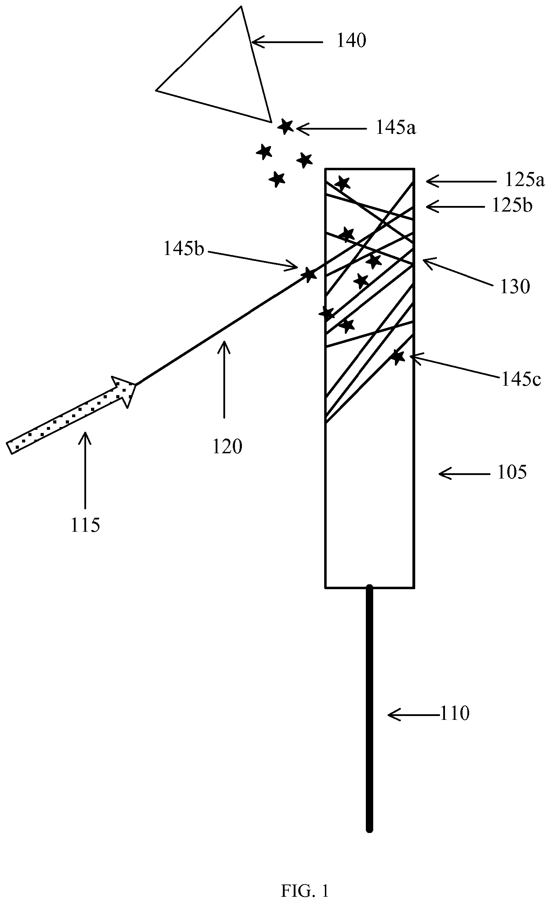

FIG. 1 illustrates one example of a system for electrospinning a polymer nanofiber onto a mandrel to form a polymer network. A polymer injection device **115** may express the polymer solution in drops or in a continuous stream. An electrospun polymer nanofiber **120** may form during the transit of the liquid polymer from the injector **115** to the mandrel **105.** A voltage source may provide a high voltage to the injection device **115** with an appropriate ground to the mandrel **105.** The mandrel **105** may be mounted on a movable support **110.** In one non-limiting embodiment, the high voltage ground may be in electrical communication with the support **110.** In another non-limiting embodiment, the high voltage ground may be in electrical communication with the mandrel **105.**

In one non-limiting example, the support **110** may cause the mandrel **105** to rotate either in a single direction during the electrospinning process, or in alternating directions. In an alternative non-limiting example, the support **110** may cause the mandrel **105** to translate along one or more linear axes, x, y and/or z during the electrospinning process, or in alternating directions. Such linear motions may permit the fiber **120** to attach to any portion along the length of the mandrel **105** (*viz.* in the z-direction). Alternatively, liner motions may cause the mandrel **105** to vary in its distance from the tip of the injection device **115** (x- and/or y-directions). It may be understood that the support **110** may move the mandrel **105** in a complex motion including both rotational and translational motions during the electrospinning process. It may further be appreciated that the speed of rotation and/or translation of the mandrel **105** during the electrospinning process may be uniform or nonuniform. It may also be appreciated that the mandrel **105** on the support **110** may be static and that the injection device **115** may rotate and/or translate with respect to the static mandrel.

As illustrated in FIG. 1, the electrospun fiber **120** may be wound about the mandrel **105** in a manner similar to that used to wind spun thread on a bobbin. The fiber **120** may be wound in any number of controlled configurations about the mandrel **105** based, at least in part, on one or more factors including the rate of polymer solution injection by the injection device **115**, the voltage potential between the injection device and the mandrel, and the rotational and/or translation speed of the support **110.** The mandrel **105** may have any shape appropriate to the type of luminal structure intended for manufacture. In one non-limiting example, the mandrel **105** may have a simple tubular shape, for example, for a vascular support structure. In another non-limiting example, the mandrel **105** may be composed of a structure having a single tubular end, and a bifurcated tubular end. It may be apparent that such a shape may be used to fabricate a polymer network appropriate to replace a trachea and attendant bronchi. In one non-limiting example, the mandrel **105** may be composed of metal. In another non-limiting example, the mandrel **105** may be coated with a non-stick material, such as, for example, aluminum foil or polytetrafluoroethylene, to permit easy removal of the polymer network from the mandrel. In still another non-limiting embodiment, the mandrel **105** may have a collapsible construction, so that the mandrel may be removed from the polymer network by collapsing the mandrel within the polymer network, thereby freeing the polymer network from the mandrel surface. The mandrel **105**, having a completed polymer network wrapped around it, may be sprayed with a solvent, such as an alcohol, to loosen the polymer network from the mandrel, thereby permitting the polymer network to be removed from the mandrel, thereby forming the biocompatible textile. A mandrel **105** having a more complex shape may be fabricated from a number of reversibly attachable components. A polymer network fabricated on such a mandrel **105** may be removed from the mandrel surface by dissembling the mandrel.

As illustrated in FIG. 1, a rotating mandrel **105** may cause the fiber **120** to wrap around the mandrel outer surface forming a plurality of windings **125a,b.** The windings **125a,b** may be formed in regular, irregular, or a combination of regular and irregular patterns. In one embodiment, the windings **125a,b** may form a regular right-handed helix. In one embodiment, the windings **125a,b** may form a regular left-handed helix. In some embodiments, the windings **125a,b** may form a helix with about the same spacing between adjacent windings. In some embodiments, the windings **125a,b** may form a helix in which adjacent windings have different spacing between them. In some additional embodiments, the windings **125a,b** may not form a regular helix, and there may be overlap among some number of windings. In still another embodiment, the windings **125a,b** may be wound around the mandrel **105** in a random manner.

It may be apparent that the polymer network may be composed of a number of windings **125a,b.** The polymer network may be composed of a single layer of windings **125a,b.** In alternative embodiments, the polymer network may be composed of a number of layers of windings **125a,b.** In some embodiments, a number individual fibers **120** may be wound consecutively around the mandrel **105** to form one or more layers. In alternative embodiments, each layer may be composed of a number of windings **125a,b** of a single fiber **120.** In still alternative embodiments, a number of layers may be composed of a single fiber **120**, wound around the mandrel **105** in a succession of layers. It may be appreciated that a void or inter-fiber spacing **130** may be formed between adjacent windings, such as between winding **125a** and **125b.** Such inter-fiber spacings **130** may have a diameter of about 2 microns to about 5 microns. Alternatively, such inter-fiber spacings **130** may have a diameter of about 30 micron to about 50 microns. It may be apparent that a textile may include a plurality of inter-fiber spacings **130** having a diameter of about 2 microns to about 50 microns. Non-limiting examples of such inter-fiber spacings may include about 2 µm, about 4 µm, about 6 µm, about 8 µm, about 10 µm, about 20 µm, about 30 µm, about 40 µm, about 50 µm, or ranges between any two of these values (including endpoints). In additional non-limiting examples, the inter-fiber spacings **130** may have a diameter less than or about equal to about 200 microns. The textile may alternatively be characterized by a mesh size. In some non-limiting embodiments, the textile may have a mesh size of about 20 inter-fiber spacings **130** per mm to about 500 inter-fiber spacings **130** per mm. Non-limiting examples of such mesh sizes may include about 20 spacings per mm, about 40 spacings per mm, about 60 spacings per mm, about 80 spacings per mm, about 100 spacings per mm, about 200 spacings per mm, about 300 spacings per mm, about 400 spacings per mm, about 500 spacings per mm, or ranges between any two of these values (including endpoints).

An alternative method for fabricating a biocompatible polymer textile may include surface treatments to further adjust the mesh size of the polymer network. A surface treatment may include contacting particulate material **145a** with one or more of the electrospun fiber **120**, the mandrel **105**, or the polymer network disposed on the mandrel. In one non-limiting method, a particulate material **145b** may be contacted with the electrospun fiber **120** during the spinning step (**145b**) before the electrospun nanofiber contacts the mandrel **105.** In an alternative method, the particulate material **145c** may be contacted with a polymer network of electrospun fibers (**145c**) in contact with the mandrel **105.** The particulate material **145a** may be supplied from a particulate source **140** placed above or otherwise proximate to the electrospun fiber **120** or mandrel **105.** The particulate source **140** may include any device known in the art including, without limitation, a sieve or a shaker. The particulate source **140** may be mechanical in nature and may be controlled by an operator directly, a mechanical controller, an electrical controller, or any combination thereof.

The particulate material **145a** may be chosen from any material capable of being dissolved in a solvent that may not otherwise dissolve the electrospun fibers. In one non-limiting example, the solvent may be water, and the particulate material **145a** may include water-soluble particulates. Water-soluble particulates may include a water-soluble salt, a water-soluble sugar, a hydrogel, or combinations thereof. Non-limiting examples of a water-soluble salt may include NaCl, CaCl, CaSO₄, or KCl. Non-limiting examples of water-soluble sugars may include sucrose, glucose, or lactose. The particulate material **145a** may have an average size of about 5 µm to about 3000 µm. Non-limiting examples of the average size of such particulate material **145a** may include about 5 µm, about 10 µm, about 50 µm, about 100 µm, about 500 µm, about 1000 µm, about 2000 µm, about 3000 µm, or ranges between any two of these values (including endpoints).

Fabrication methods of multi-layer textiles may include additional steps. In some examples, groups of layers may be tack welded together either chemically or thermally. Alternatively, layers may be sintered together either through thermal or chemical means.

As disclosed above, a textile may be composed of a number of layers of windings 225. Additional features may be added to the textile. The textile may include one or more curved support structures. Such structures may include rings or U-shaped supports disposed on the interior of the textile, exterior of the textile, or between successive layers of fiber windings **125a,b.** The curved supports may have any dimensions appropriate for their use. The supports may have a width of about 0.2 mm to about 3 mm. The supports may have a thickness of about 0.2 mm to about 3 mm. Such supports may be composed of one or more of the following: metals, ceramics, and polymers. Non-limiting examples of polymers used in such curved supports may include: a polyethylene terephthalate, a polyester, a polymethylmethacrylate, polyacrylonitrile, a silicone, a polyurethane, a polycarbonate, a polyether ketone ketone, a polyether ether ketone, a polyether imide, a polyamide, a polystyrene, a polyether sulfone, a polysulfone, a polycaprolactone (PCL), a polylactic acid (PLA), a polyglycolic acid (PGA), a polyglycerol sebacic, a polydiol citrate, a polyhydroxy butyrate, a polyether amide, a polydiaxanone, a chitosan, and combinations or derivatives thereof. Such support may further be composed of materials that may be non-resorbable or fully degradable. Such supports may be affixed on the mandrel **105** before the fiber **120** is wound about the mandrel surface to form the polymer network. Alternatively, such supports may be affixed on one pre-wound layer of fiber **120** of the polymer network before additional layers are wound on top of it. Such supports may be attached to the outer surface of the polymer network after the winding process is complete but before the polymer network is removed from the mandrel **105.** Alternatively, after the polymer network has been removed from the mandrel **105**, thereby forming the biocompatible textile, additional supports may be added. Supports may be affixed on the polymer network or textile by any appropriate means, including, but not limited to, gluing, heat welding, and solvent welding. Additional supports may be formed of a mesh material over which the fibers **120** may be spun. Such a mesh support may be rigid, collapsible, and/or expandable.

The polymer network may receive any of number of additional surface treatments while still in contact with the receiving surface. Alternatively, once the polymer network has been removed from the receiving surface, thereby forming the implantable biocompatible textile, such surface treatments may also be applied. Non-limiting examples of such surface treatments may include, without limitation, washing in a solvent, drying with a gas stream, sterilizing, sintering, or treating with a plasma discharge. Washing solvents may include water and alcohol. A drying gas stream may include air, nitrogen, or inert gas such as argon. A gas stream may include pressurized air or pressurized ionized air. Sterilization techniques may include gamma irradiation and electron beam. Plasma discharge treatments may be performed in air or in another gas such as carbon tetrafluoride.

The disclosure above includes a number of embodiments and examples of polymer networks and biocompatible textiles fabricated by electrospinning a polymer to form windings about a mandrel. Such windings may be fabricated by rotating the mandrel about a longitudinal axis and contacting the electrospun polymer nanofiber with the mandrel. During the winding process, the mandrel additionally may be translated in any of a number of directions (in an x-direction, a y-direction, and a z-direction) with respect to the polymer injection device while simultaneously rotating about its longitudinal axis. Alternatively, the polymer networks or biocompatible textiles may be fabricated by translating the mandrel along a longitudinal axis to receive the electrospun polymer and rotating after a translation step has been accomplished. The resulting polymer network structure may not include windings (that is, a fiber deposited on the mandrel in a circular or curved path about the mandrel longitudinal axis) but rather may be composed of overlaid linear threads of fibers deposited on the mandrel surface along the longitudinal axis of the mandrel.

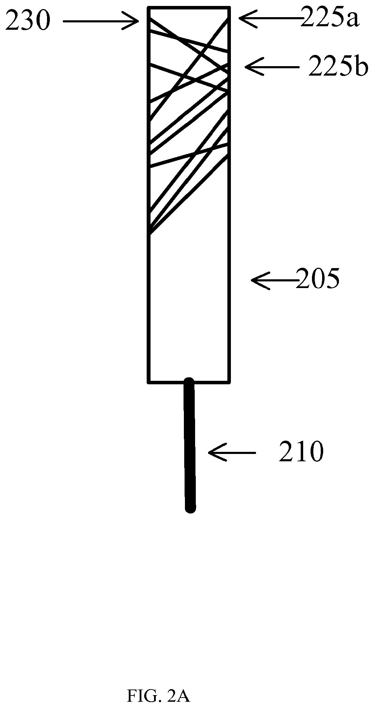

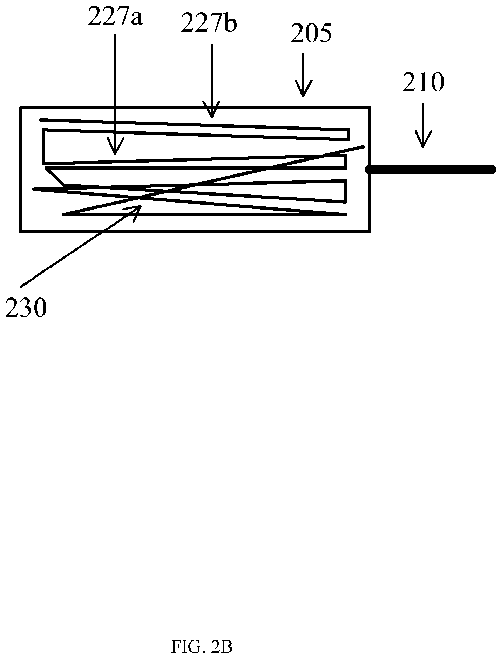

FIGS. 2A and 2B illustrate some examples of these geometries. As illustrated in FIG. 2A, mandrel **205** on a support **210** may rotate about the longitudinal axis of the mandrel. The electrospun polymer may be contacted with the mandrel **205** while the mandrel rotates, thereby producing windings **225a, 225b** about the longitudinal axis of the mandrel with a spacing **230** therebetween. As illustrated in FIG. 2B, the mandrel **205** on the support **210** may be linearly translated while the polymer nanofiber contacts the mandrel surface. As a result, the electrospun nanofibers may form one or more textile threads **227a,b** having a primarily longitudinal orientation along the mandrel longitudinal axis. These textile threads **227a,b** may also be characterized by an inter-fiber spacing **230.** It may be appreciated that polymer networks or biocompatible textiles having a variety of fiber and/or winding orientations and spaces may be fabricated based on the direction and orientation of mandrel motion with respect to the polymer injector.

It may be understood that in the above disclosure, the use of a mandrel as a polymer receiving surface is not considered limiting. Other types of surfaces, including planar surfaces, circular surfaces, and irregular surfaces, may equally be used as receiving surfaces for the polymer electrospun fibers. Rotational and translational motions of such alternative receiving surfaces may result in any type of orientation of polymer nanofiber threads on or about the receiving surface. Thus, such polymer nanofiber threads may form, without limitation, windings, linear alignments, star-shaped alignments, or random alignments.

### Biocompatibility

In certain instances, textiles can be manufactured that lack openings between the fibers, and may be considered "meshless" textiles. In such "meshless" textiles, the fibers may be too big to allow openings between neighboring fibers. Such fibers in "meshless" textiles may almost entirely overlap each other, thereby creating an effectively continuous textile surface. Pores, including, without limitation, pockets, spaces, voids, and holes, may be introduced into such "meshless" textiles. Those having skill in the art may recognize that, as one non-limiting example, methods capable of incorporating pores into a polymer block may also be used to introduce pores into "meshless" textiles. An artisan having average skill in the art may recognize that techniques used for introducing pores into a construct fabricated from a polymer may include, without limitation, solution casting, emulsion casting, polymer blending, and phase transition techniques.

Implants and methods that lead to patient death, or fail to delay, prevent, or mitigate morbidity and mortality, are not within the meaning of the term "biocompatible." As such, in some embodiments, ineffective, toxic, or deadly compositions are expressly disclaimed herein as they do not meet the necessary requirement of being biocompatible. In some embodiments, non-biocompatible compositions that are specifically disclaimed are: molded nanocomposites, molded polylactic acid (PLA), molded polyglycolic acid (PGA), molded polycaprolactone (PCL), polycaprolactone/polycarbonate (80:20%) polyhedral oligomeric silsesquioxane; polyhedral oligomer silsesquioxane nanocages; molded protein materials, molded collagen; molded fibrin, molded polysaccharides molded chitosan, molded glycosaminoglycans (GAGs); molded hyaluronic acid, a set nanocomposite material composed of polyhedral oligomeric silsesquioxane (POSS) covalently bonded to poly(carbonate-urea)urethane (PCU) to form a nanocomposite "POSS-PCU" polymer; a POSS-PCU fluid; coagulated POSS-PCU fluid; a POSS-PCU polymer fluid comprising salt crystals; a coagulated POSS-PCU polymer wherein the salt crystals are dissolved after coagulation to form pores; a coagulated POSS-PCU polymer wherein the salt crystals are a sodium salt, a lithium salt, a potassium salt, carbonate or bicarbonate salt, calcium carbonate, cobalt(II) carbonate, copper(II) carbonate, lanthanum carbonate, lead carbonate, lithium carbonate, magnesium carbonate, manganese(II) carbonate, nickel(II) carbonate, potassium carbonate or sodium carbonate; a POSS-PCU polymer wherein the average pore diameter may is about 20-100 microns; a molded polymer wherein the average pore diameter is about 20-100 microns, from about 1 nm to about 500 microns, an average diameter of about 10 nm to about 1 micron, about 1 to about 10 microns, about 10 to about 100 microns, about 10 to about 50 microns, about 50 to about 100 microns, about 100 to about 200 microns, about 200 to about 500 microns, and about 50-100 microns; and a polymer fluid containing 50% sodium bicarbonate having an average crystal size of about 40 microns.

### Surgical procedures

Described herein are other methods including surgical methods which can be easily adapted for use with the disclosed textile implants. For example, a subject may be evaluated using one or more imaging techniques to identify the location and extent of damaged tissue that needs to be removed or repaired. In some non-limiting examples, the disclosed textiles may be seeded on both external and luminal surfaces with compatible cells that retain at least some ability to differentiate. The cells may be autologous cells that may be isolated from the patient (e.g., from the patient bone marrow) or allogeneic cells that may be isolated from a compatible donor. The seeding process may take place in a bioreactor (e.g., a rotating bioreactor) for a few weeks, days, or hours prior to surgery. Additionally, cells may be applied to the biocompatible textile immediately before implantation. Just prior to surgery, additional cells may be added to the luminal surface of a synthetic tissue composed of a biocompatible textile. these cells may be epithelial cells, which may be isolated from the patient's airway if the tissue is an airway tissue. Additionally, one or more growth factors may be added to the synthetic tissue immediately prior to surgery. The biocompatible textile incorporating such cells and/or additional chemical factors may then be transplanted into the patient to replace the damaged tissue that has been removed. The patient may be monitored post-surgically for signs of rejection or of a poorly functional airway transplant. It should be appreciated that the addition of cells and/or chemical factors to the biocompatible textile may not be required for every transplant surgery. Any one or more of these procedures may be useful alone or in combination to assist in the preparation and/or transplantation of a synthetic organ or tissue.

The biocompatible textiles described herein may be used as a tracheal implant. The natural trachea is a cartilaginous and membranous tube that extends from the lower part of the larynx (at the level of the sixth cervical vertebra) to the upper border of the fifth thoracic veltebra, where it branches to form the two bronchi. The trachea has the shape of a cylinder that is flattened at the back (posterior). The front (anterior) is convex. Without intending to be bound, a typical adult human trachea is at least about 10 cm long, and about 2-2.5 cm wide. However, it is generally larger in males and smaller in females. Sometimes, because of disease or trauma, a patient would benefit from having support or replacement of their airway or a portion thereof (e.g., a trachea or portion thereof, a bronchus or portion thereof, or a combination thereof) with a biocompatible textile.

Any airway implant, whether comprising molded implants or textile implants, may be shaped or formed to represent the region or regions of the airway that is being replaced. The airway implant may be roughly cylindrical, thereby forming an air flow conduit after implantation. Alternatively, the air flow conduit can have the natural shape of an airway region. For example, in cross-section, the conduit may have a D shape with a convex anterior (e.g., U-shaped) and a relatively straight posterior. The length of the conduit can be designed to effectively match (or be slightly longer than) the length of the airway region being replaced. The air flow conduit can be modeled on the portion of the patient's tissue that is to be replaced by the implant. Accordingly, the dimensions and shape of the implant can be designed to match those of the airway region being replaced. It should be appreciated that, depending on the region that is being replaced, the overall shape of the conduit may be a straight conduit, a Y-shaped bifurcated conduit, an L-shaped conduit, or simply a patch applied to the existing airway.

As to care of a patient after implantation, certain biological functions may be monitored. For example, after implantation of a synthetic or natural trachea, patient follow-up may include, but is not limited to, endoscopic evaluation (flexible and/or rigid bronchoscopy) of the transplanted airway every day for the first week, every other day for the second week, once a month for the first six months thereafter, and every 6 months for the first 5 years thereafter. Additional patient follow-up may include an evaluation of the blood count, including white blood cell differentials, every second day for the first two weeks, evaluation of the hematopoietic stem cells, immunogenic evaluations (for example, a blood sample may be taken to study histocompatibility of the implant by evaluating the antibodies after 3 days , 7 days, 30 days, 3 months, 6 months, and 12 months after the transplant,), and computerized tomography of the neck and chest. Longer term patient follow-up for a transplanted airway can be performed at month 1, month 3, and month 6 of the follow-up, and every 6 months thereafter for the first 5 years. Additional oncological follow-up will be life-long and may include the standard evaluations for such medical condition. In certain embodiments, a biocompatible textile implant may be retained within the patient for 1 day, 3 days, 5 days, 7 days, 2 weeks, 3 weeks, or even a month or longer after implantation. Thus, such textiles can be retained in the patient for at least these lengths of time commensurate with biocompatibility of the implant as disclosed herein. A biocompatible textile implant may be retained in a patient for 6 month, a year, a term of years, or even as long as the lifetime of the patient.

Although disclosed above are examples of the use of a biocompatible textile to replace tracheae or laryngeal structures, it may be appreciated that other biological structures, tissues, and organs having a luminal structure may also be replaced or repaired by biocompatible textile devices. Some non-limiting examples of such luminal structures may include a trachea, a trachea and at least a portion of at least one bronchus, a trachea and at least a portion of a larynx, a larynx, an esophagus, a large intestine, a small intestine, an upper bowel, a lower bowel, a vascular structure, a nerve conduit, and portions thereof.

### Electrospun sutures

In addition to electrospun textiles, electrospun fibers may also be used singly or in bundles to form sutures. Electrospun fiber sutures may have a length of about 1 cm to about 50 cm. Electrospun sutures may be required to meet or ideally exceed industry standard properties such as those disclosed by the United States Pharmacopeia. Table 1 discloses standard measures required for synthetic, absorbable sutures according to the U.S. Pharmacopeia. It may be appreciated that electrospun sutures may be either resorbable or non-resorbable. Additionally, electrospun sutures may incorporate or be coated with biologically active materials. Such biologically active materials may include antibiotics, cell growth factors, anti-coagulant factors, or any combination thereof. In one non-limiting example, electrospun nanofiber sutures may incorporate antibacterial agents that may diffuse into the surrounding tissue, thereby reducing or preventing local infections at the suture sites. Examples of such antibiotic agents may include, without limitation, penicillins, quinolones and tetracyclines. Non-limiting examples of growth factors and biologics may include nerve growth factor, vascular endothelial growth factor, and platelet-rich plasma. In addition, viruses such as a retrovirus including lentivirus for gene-therapy purposes, micro RNA, and small molecules may be added to the electrospun fibers.

**Table 1**

| USP Size | Size (Metric Gauge) | Min. Diameter (µm) | Max. Diameter (µm) | Knot-Pull Tensile Strength (N) |
|---|---|---|---|---|
| 12-0 | 0.01 | 1 | 9 | N/A |
| 11-0 | 0.1 | 10 | 19 | N/A |
| 10-0 | 0.2 | 20 | 29 | 0.24 |
| 9-0 | 0.3 | 30 | 39 | 0.49 |
| 8-0 | 0.4 | 40 | 49 | 0.69 |
| 7-0 | 0.5 | 50 | 69 | 1.37 |
| 6-0 | 0.7 | 70 | 99 | 2.45 |
| 5-0 | 1 | 100 | 149 | 6.67 |
| 4-0 | 1.5 | 150 | 199 | 9.32 |
| 3-0 | 2 | 200 | 249 | 17.4 |
| 2-0 | 3 | 300 | 339 | 26.3 |

Single stranded electrospun fiber sutures may be used as produced or may also be twisted to improve their strength. In some non-limiting examples, twisted electrospun fibers may have a twist of about 0 twists per meter ("TPM") to about 5000 twists per meter (TPM). In one non-limiting example, a twisted electrospun fiber may have a twist of about 5000 twists per meter (TPM). Bundles of electrospun fibers, composed of 3 to 10,000 fibers may be twisted or braided together for improved properties. Sutures composed of bundles of electrospun fibers may also be composed of bundles of non-twisted or twisted electrospun fibers. Fiber bundles may be coated with lubricous compounds to improve hand ability or to reduce the surface roughness. Fiber bundles may also be heat treated to relieve mechanical stresses from braiding and twisting or to help align the polymer chains and increase the crystallinity to improve the mechanical strength.

### Micronized Electrospun Textile

In addition to electrospun textiles and sutures, biocompatible electrospun nanofiber textiles may also be micronized. Such micronized textiles may be prepared by freezing an electrospun textile, for example in liquid nitrogen. Freezing the electrospun fibers may result in increased brittleness, resulting in textiles that may be readily pulverized into small fragments. Pulverization techniques may include, without limitation, grinding, chopping, pulverizing, micronizing, milling, shearing, or any combination thereof. Fragments may have an average length of about 10 µm to about 1000 µm. In one non-limiting example, fragments may have an average length of about 100 µm. Such micronized textiles may also be compressed into fiber suspensions. In one non-limiting example, the compressed fiber suspension may be pelletized, or otherwise formed into a compressed or pellet-like structure.

Such micronized electrospun fibers may be added to a carrier medium to produce a suspension for injection into a body part. The suspension for injection may have a volume of about 0.1 mL to about 50 mL. The suspension may also comprise micronized electrospun fiber fragments in a weight percent to carrier medium of about 0.001 wt% to about 50 wt%. In some non-limiting examples, the carrier medium may be any type of medium, including pastes, liquids, gels, aerosols, powders, and the like. In some non-limiting examples, the carrier medium may be phosphate buffered saline, cell culture media, platelet-rich plasma, plasma, lactated Ringer's solution, a gel, or any combination thereof. In some non-limiting examples, the suspension may be injected into a joint. Non-limiting examples of joints in which the suspension may be injected may include the knee, the shoulder, or the hip. In one non-limiting example, the suspension may be injected using a syringe with a 20-gauge needle.

A localized injection of a suspension of micronized electrospun fibers may be useful for repair of joint structures, such as a knee meniscus. Alternatively, such a suspension may be used to reduce local joint inflammation, such as inflammation caused by arthritis. An injection of micronized electrospun textile fragments may be used to repair localized tissue injuries such as muscle tears, ligament tears, and tendon tears. Muscle injuries that may be repaired by such a suspension may include injuries to striated muscle, smooth muscle, and cardiac muscle. It may be appreciated that such micronized electrospun fiber fragments may be used for such purposes in humans as well as in non-human animals (veterinary applications).

Suspensions of micronized electrospun fiber fragments may include additional components along with the carrier medium. Non-limiting examples of additional bioactive components may include antibiotics, drugs, tissue growth factors, platelet-rich plasma, amnion, small molecules, or any combination thereof. Biologically active cells may also be included in the suspensions. Biologically active cells may include differentiated cells, stem cell, or any combination thereof. Such biologically active cells may be added to the suspensions to provide cells for improved repair of injured tissues. Stem cells may include multipotent stem cells, pluripotent stem cells, and totipotent stem cells. Such stem cells may be autologous (from the same patient), syngeneic (from an identical twin, if available), allogeneic (from a non-patient donor), or any combination thereof. In some non-limiting embodiments, the stem cells may include adult stem cells such as bone marrow-derived stem cells, cord blood stem cells, or mesenchymal cells. Other types of stem cell may include embryonic stem cells or induced pluripotent stem cells. It may be appreciated that a suspension of micronized electrospun fiber fragments in a carrier fluid may incorporate adult stem cells, embryonic stem, induced pluripotent stem cells, differentiated cells, or any combination thereof.

Micronized nanofiber textile fragments may be combined with other carrier materials and are not limited to purely aqueous suspensions. Micronized nanofiber textile fragments may be combined with gels, pastes, powders, aerosols, and/or other carriers. In one non-limiting example, the textile fragments may be combined with a carrier capable of forming a gel or solid when injected into a recipient (human or non-human animal). Gelation or solidification of the carrier may occur on exposure of the suspension to the biological environment due, for example, to a change in temperature or pH. Alternative carriers may include components capable of responding to externally applied stimuli such as magnetic fields, electric fields, or sonic fields. In one non-limiting example, a carrier may respond to an applied magnetic field to cause the textile fragments to orient in a specific direction. In one non-limiting application, micronized nanofiber textile fragments may be implanted directly into a solid tumor. The implanted textile fragments may concentrate externally applied heat, sonic, or radiation energy to the tumor.

### Electrospun Nanofiber Fragments and Clusters

In addition to micronized biocompatible electrospun nanofiber textiles, nanofibers may also be processed into small fragments and aggregates of fragments, or clusters. Such fragments or clusters may be initially prepared by the processes described herein, followed by one or a range of pulverizing procedures, as described above. Such fragments or clusters may be either resorbable or non-resorbable, or a combination thereof. Fragments may have an average length of about 10 µm to about 1000 µm. In one non-limiting example, fragments may have an average length of about 100 µm. Clusters may have a range of shapes. Non-limiting examples of cluster shapes include spherical, globular, ellipsoidal, and flattened cylinder shapes. Clusters may have, independently, an average length of about 1 µm to about 1000 µm, an average width of about 1 µm to about 1000 µm, and an average height of about 1 µm to about 1000 µm, and may include an average number of about 2 to about 1000 fiber fragments. In one non-limiting example, clusters may include an average number of about 100 fiber fragments. In some embodiments, the electrospun nanofiber fragments and/or clusters may be used to retain or localize cells or other components incorporated therewith, to promote cell infusion, attachment, adhesion, penetration, or proliferation, to stimulate cell or tissue growth, healing, or, in some cases, shrinkage, or any combination of uses thereof.

Such electrospun nanofiber fragments and/or clusters may be fabricated from a polymer solution, as described above. The polymer solution may include additional materials. In a non-limiting example, electrospun nanofiber fragments and/or clusters may be manufactured or impregnated with additional materials, which the fragments and/or clusters may later elute. Non-limiting examples of such additional materials may include radiation opaque materials, electrically conductive materials, fluorescent materials, luminescent materials, antibiotics, growth factors, vitamins, cytokines, steroids, anti-inflammatory drugs, small molecules, sugars, salts, peptides, proteins, cell factors, DNA, RNA, any materials to aid in non-invasive imaging, or any combination thereof. Non-limiting examples of radiation opaque materials may include barium, tantalum, tungsten, iodine, or gadolinium. Non-limiting examples of electrically conductive materials may include gold, silver, iron, or polyaniline.

A composition or kit of the invention comprising such electrospun nanofiber fragments and clusters comprises a carrier medium to produce a suspension for delivery to a body part or system. The suspension may have a volume of about 0.1 mL to about 50 mL. The suspension may also comprise electrospun nanofiber fragments and/or clusters in a weight percent to carrier medium of about 0.001 wt% to about 50 wt%. In some non-limiting examples, the carrier medium may be phosphate buffered saline, cell culture media, platelet-rich plasma, plasma, lactated Ringer's solution, a gel, a powder, an aerosol, or any combination thereof. In some non-limiting examples, the suspension may be injected into a joint. Non-limiting examples of joints in which the suspension may be injected may include the knee, the shoulder, and the hip. In one non-limiting example, the suspension may be injected using a syringe with a 20-gauge needle. In some non-limiting examples, the suspension may be injected into a tendon or ligament. In some non-limiting examples, the suspension may be injected intravenously, intramuscularly, subcutaneously, or intraperintoneally. In some non-limiting examples, the suspension may be delivered topically. In one non-limiting example, the suspension may be applied topically to a wound. In some non-limiting examples, the suspension may be inserted during surgery. In some non-limiting examples, the suspension may be delivered by ingestion, inhalation, or suppository. In some non-limiting examples, the suspension may be printed into a construct, or scaffold. In one non-limiting example, the suspension may be printed, such as via a three-dimensional printer, for eventual application in the body or a system.

A localized injection of a suspension of electrospun nanofiber fragments andr clusters may be useful for repair of joint structures, such as a knee meniscus, cruciate ligament, or articular cartilage. Alternatively, such a suspension may be used to reduce local joint inflammation, such as inflammation caused by arthritis. In some alternative embodiments, a therapeutically effective compound may be loaded onto or incorporated into the electrospun nanofiber fragments and/or clusters themselves. In some alternative embodiments, an injection of electrospun nanofiber fragments and clusters may be used to repair localized tissue injuries such as muscle tears, ligament tears, and tendon tears. Muscle injuries that may be repaired by such a suspension may include injuries to striated muscle, smooth muscle, and cardiac muscle. It may be appreciated that such electrospun nanofiber fragments and/or clusters may be used for such purposes in humans as well as in non-human animals, such as for veterinary applications.

A localized injection of a suspension of electrospun nanofiber fragments and clusters may also be used to fill voids, such as those found beneath skin wrinkles. Alternatively, such a suspension could be used to fill voids, such as sphincter voids associated with anal, colon, urinary, or other types of incontinence. Such applications may be used, for example, for medical, treatment, cosmetic, aesthetic, or any other purpose or combination of purposes. In some alternative embodiments, a localized injection of such a suspension could be used as a bulking agent in muscles. In some alternative embodiments, a localized injection of such a suspension could be used as an anti-wrinkle agent injected beneath the skin.

A localized injection of a suspension of electrospun nanofiber fragments and clusters may also be used as an embolization agent, such as in association with an aneurysm. In one non-limiting example, a localized injection of such a suspension, combined or otherwise added to platelet-rich plasma, may be used to occlude an aneurysm of any blood vessel, including those of the brain, heart, and other major organs.

A suspension of electrospun nanofiber fragments and clusters may also be used as a material on which or in which cells may incubate, adhere, grow, proliferate, and/or differentiate, as opposed to combining previously grown or expanded cells with a previously created suspension of electrospun nanofiber fragments and clusters. In a non-limiting example, a suspension of electrospun nanofiber fragments and clusters may be used as a material for the incubation, growth, proliferation, and/or differentiation of cells *in vitro*, followed by injection or implantation *in vivo*, as opposed to growing cells on polymer microcarriers, releasing the cells from the microcarriers, separating the cells from the microcarriers, and then implanting the cells *in vivo.* In some embodiments, such an application would reduce or eliminate the need to process cells between culture and implantation, thereby improving cell yield and reducing waste of any cells or materials used in cell growth or proliferation.

The above-described suspensions of electrospun nanofiber fragments and clusters may include additional components along with the carrier medium. Non-limiting examples of additional bioactive components may include antibiotics, tissue growth factors, platelet-rich plasma, amnion, small molecules, or any combination thereof. Biologically active cells may also be included in the suspensions. Biologically active cells may include differentiated cells, stem cells, or any combination thereof. Such biologically active cells may be added to the suspensions to provide cells for improved repair of injured or stunted tissues. Stem cells may include multipotent stem cells, pluripotent stem cells, and totipotent stem cells. Such stem cells may be autologous (from the same patient), syngeneic (from an identical twin, if available), allogeneic (from a non-patient donor), or any combination thereof. In some non-limiting embodiments, the stem cells may include adult stem cells such as bone marrow-derived stem cells, cord blood stem cells, or mesenchymal cells. Other types of stem cells may include embryonic stem cells or induced pluripotent stem cells. It may be appreciated that a suspension of electrospun nanofiber fragments and/or clusters in a carrier medium may incorporate adult stem cells, embryonic stem, induced pluripotent stem cells, differentiated cells, or any combination thereof.

Electrospun nanofiber fragments and clusters may be combined with other carrier materials, and are not limited to purely aqueous suspensions. In some other non-limiting embodiments, micronized nanofiber textile fragments may be combined with gels, pastes, powders, aerosols, and/or other carriers. In one non-limiting example, the nanofiber fragments and/or clusters may be combined with a carrier capable of forming a gel, solid, powder, or aerosol when implanted into a recipient (human or non-human animal). Gelation or solidification of the carrier may occur on exposure of the suspension to the biological environment due, for example, to a change in temperature or pH. Alternative carriers may include components capable of responding to externally applied stimuli such as magnetic fields, electric fields, or sonic fields. In one non-limiting example, a carrier may respond to an applied magnetic field to cause the textile fragments to orient in a specific direction. Electrospun nanofiber fragments and/or clusters without a carrier may also be implanted in a recipient. In one non-limiting application, electrospun nanofiber fragments and/or clusters may be implanted directly into a solid tumor. The implanted fragments and/or clusters may concentrate externally applied heat, sonic, or radiation energy to the tumor. In one non-limiting example, electrospun nanofiber fragments and/or clusters may be implanted for the purpose of localized or systemic delivery of drugs, biological materials, contrast agents, or other materials, as disclosed above.

In one non-limiting example, electrospun nanofiber fragments and clusters may be sold in a kit. The kit further comprises a carrier medium. In a non-limiting example, the kit may further comprise instructions for the use of the electrospun nanofiber fragments, clusters, and/or carrier medium. In a non-limiting example, the carrier medium may be any of the above-disclosed carrier media, in any form, including, for example, a gel, a dry form such as a powder, an aerosol, a liquid, or any other form, including those which may be reconstituted for use.

In order to illustrate the various features disclosed above, the following non-limiting examples are provided.

### EXAMPLES

### Example 1: Method of Preparing a Biocompatible Textile

In preparing an exemplary textile, a polymer nanofiber precursor solution was prepared by dissolving 2-30 wt% polyethylene terephthalate (PET) in a mixture of 1,1,1,3,3,3-hexafluoroisopropanol and trifluoroacetic acid, and the solution was heated to 60°C followed by continuous stirring to dissolve the PET. The solution was cooled to room temperature and the solution placed in a syringe with a blunt tip needle. The nanofibers were formed by electrospinning using a high voltage DC power supply set to 1kV-40kV positive or negative polarity, a 5-30 cm tip-to-substrate distance, and a 1µl/hr to about 100 mL/hr flow rate from the syringe. It is possible to use a needle array of up to 1,000's of needles to increase output. An approximately 0.2 -3.0 mm thickness of randomly oriented and/or highly-aligned fibers were deposited onto a receiving surface. Polymer rings were introduced onto the receiving surface and over the previously spun fibers, and an additional approximately 0.2-3.0 mm of fiber was added over the surface (including the additional polymer rings) while the form was rotated. The textile was placed in a vacuum overnight to ensure removal of residual solvent (typically less than 10 ppm) and treated using a radio frequency gas plasma for 1 minute to make the fibers more hydrophilic and promote cell attachment.

### Example 2: A Biocompatible Textile Fabricated with Soluble Particulate Materials

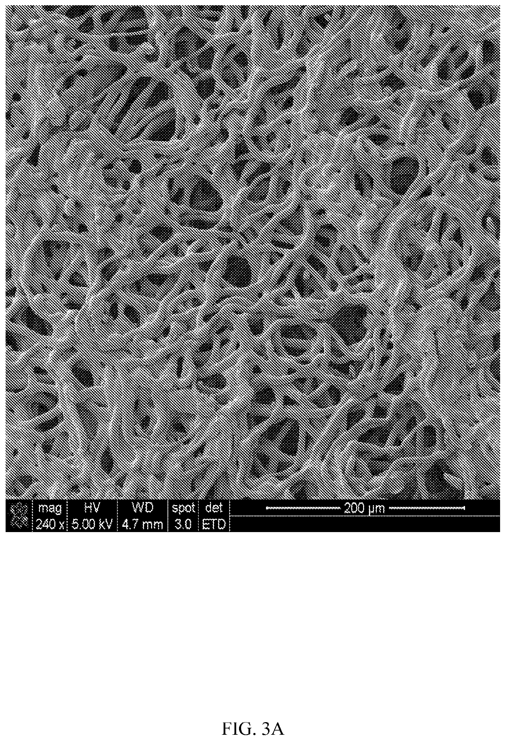

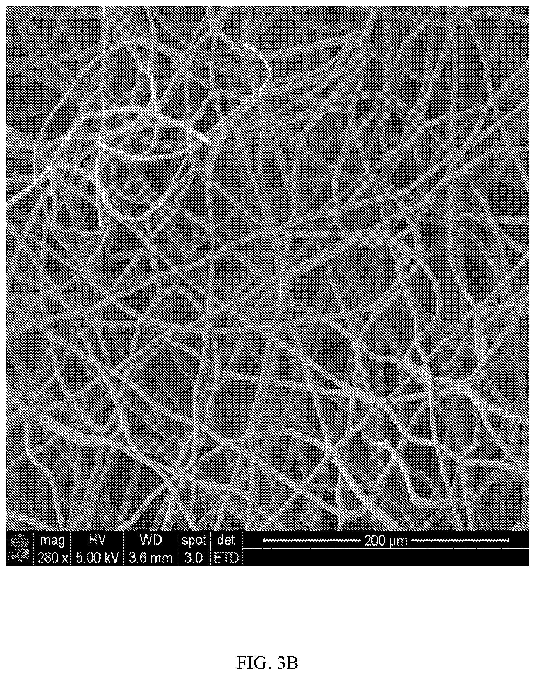

FIGS. 3A and 3B depict two micrographs of biocompatible textiles, one formed without the addition of a soluble particulate material (FIG. 3A) and one formed with the addition of the soluble particulate material (FIG. 3B). The textiles were formed from a solution of polydioaxone dissolved in hexafluoroisopropanol at a concentration of about 13 wt%. Polymer fibers having a fiber diameter of about 7 µm were electrospun onto a mandrel. FIG. 3A depicts the textile created from the polydioxanone electrospun alone onto the mandrel and demonstrates a porosity of about 60% (in which porosity may be defined as the fraction of void space in a material). It may be observed that the porosity appears inconsistent across the area depicted in FIG. 3A. A low porosity scaffold may be beneficial in applications where cellular infiltration is to be avoided or to decrease water permeability. However, a low porosity scaffold may be a disadvantage or a flaw in applications in which cellular infiltration in the scaffold may be desired. It may be appreciated that fibers bonded together as depicted in FIG. 3A may have mechanical properties that differ from those of a mesh scaffold lacking such bonding between fibers.

FIG. 3B illustrates a micrograph of a textile formed from the same polymer solution and solvent, but which further included small particulates of NaCl added to the fiber during winding, to form a polymer network including salt. The salt particles had an average size of about 50 µm. To remove the salt from the polymer network, the network was submerged in three successive changes of water under gentle agitation using a stir bar for about 24 hrs. It may be observed in FIG. 3B that the textile mesh is more regular, having an average porosity of about 90%. It may be appreciated that the addition of a soluble particulate material having a known size during the fabrication of the textile may produce a more homogeneous porosity and may also be a more reproducible process for fabricating the textiles. Reproducibility of textile properties may improve the utility of the textiles in that the textiles may be more readily fabricated to meet known specifications. In addition to improved reproducibility of the electrospinning process, the addition of soluble particulates can facilitate the customization of the mesh size for specific applications. In one non-limiting example, it may be necessary to increase the mesh size of the textile to accommodate the seeding or culturing of large groups of cells presented to the textile as cell spheroids. Such spheroids may contain hundreds of cells and may be up to several hundred microns in diameter. The larger mesh size may allow these spheroids to fit into the scaffold without destroying the cell groups.

### Example 3: Electrospun Sutures (for reference)

Several sample single stranded nanofiber sutures having zero twists per meter (0 TMP) were fabricated. The sample nanofiber sutures were prepared from the electrospun fibers.

In one non-limiting example of a process to fabricate the sutures, polymer solutions were made by dissolving polycaprolactone into hexafluoro isopropanol via rigorous stirring by a magnetic stir bar. Solutions were transferred to a 20cc syringe capped with a 20-gauge needle and loaded into a syringe pump. To create more randomly oriented sutures in a continuous process, the polymer solution was dispensed from the syringe at a rate of 5 mL/h and aimed at a rotating aluminum funnel with approximately 15 cm distance between syringe tip and the funnel edge. A voltage of +14 kV was applied to the syringe tip to initiate electrospinning, and a -4 kV voltage was applied to the funnel apparatus to attract the fibers. A smooth glass rod was placed between the syringe tip and funnel edge, and a cone of fibers was formed between the rod and the funnel. By rotating the rod as a take up, fibers from the cone twisted into a continuous rope of fibers onto the glass rod, approximately 20-50 µm in size. To end the rope, the glass rod was pulled away from the electrospinning set up.

In another non-limiting example of a process to fabricate a highly aligned suture, the polymer solution was dispensed at a rate of 1 mL/h, aimed at a large, rotating aluminum wheel at a distance of 20 cm. The wheel was covered with 6 mil gauge Teflon film to collect the fibers to be deposited. The wheel was set to rotate at 475 RPM (16 m/s). A positive voltage of +4.3 kV was applied to the syringe tip to initiate electrospinning, and a -3.4 kV voltage was applied to the wheel to attract the fibers. Fibers were collected onto the wheel in a highly aligned orientation for 90 minutes. Following their deposition, fibers were removed from the Teflon film in bundles of fibers approximately 1mm wide, then rolled into a suture for a final thickness of 20-40 µm.

In each case, sutures were cut to a length of about 10 cm.

Table 2 discloses some properties of examples of such sutures.

**Table 2**

| Sample Number | Tensile Strength (MPa) | Percent Elongation | Diameter (µm) | Break Force (N) |
|---|---|---|---|---|
| 1 | 7.79 | 29.2 | 63 | 0.024 |
| 2 | 12.4 | 8.36 | 75 | 0.055 |
| 3 | 7.42 | 22.1 | 68 | 0.027 |
| 4 | 7.69 | 43.3 | 117 | 0.083 |
| 5 | 8.83 | 25.7 | 81 | 0.047 |
| Ave. | 8.83 | 25.7 | 80.8 | 0.047 |
| Stdev. | 2.07 | 12.6 | 21.4 | 0.024 |

The single stranded electrospun suture had an average USP size of about 6-0 (metric gauge 0.7).

### Example 4: Twisted Electrospun Sutures (for reference)

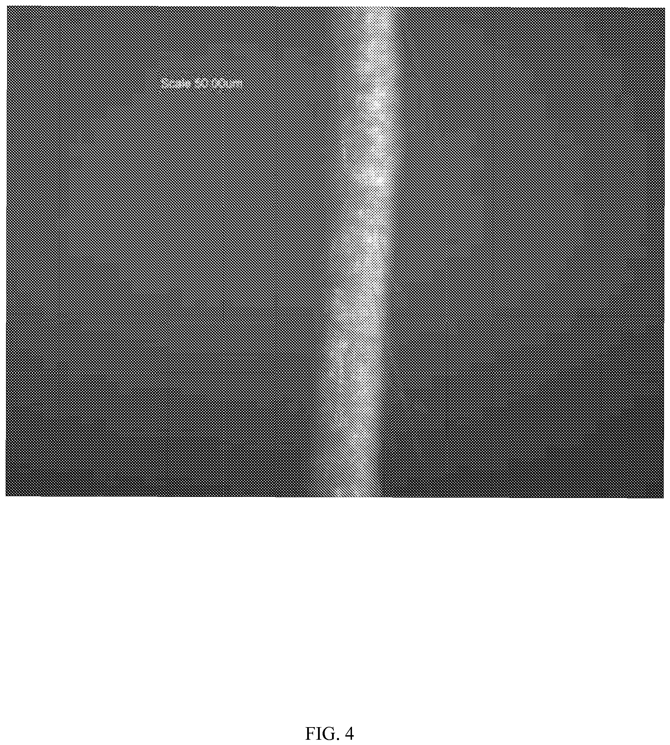

FIG. 4 depicts an image of a twisted, single strand nanofiber suture. Multiple samples were fabricated for statistical testing. The sample nanofiber sutures were prepared as disclosed in Example 3. After each sample nanofiber suture was fabricated, the fiber was twisted by attaching a suture of known length to a programmable servo motor and programming the motor to rotate the desired number of revolutions to produce a twist of about 5000 twists per meter (TPM). Table 3 discloses some properties of examples of such sutures.

**Table 3**

| Sample Number | Tensile Strength (MPa) | % Elongation | Diameter (µm) | Break Force (N) |
|---|---|---|---|---|
| 1 | 27.85 | 43.7 | 66 | 0.096 |
| 2 | 19.48 | 41.0 | 80 | 0.097 |
| 3 | 6.327 | 20.8 | 59 | 0.017 |
| 4 | 4.833 | 20.6 | 82 | 0.025 |
| 5 | 27.0 | 43.3 | 62 | 0.083 |
| Ave. | 17.1 | 33.9 | 70 | 0.064 |
| Stdev. | 11.0 | 12.1 | 10 | 0.039 |
| St. Error of the Mean | 4.93 | 5.4 | 5 | 0.018 |

The twisted single strand electrospun suture had an average USP size of about 6-0 (metric gauge 0.7). The tensile strength of the twisted sutures did not appear to increase monotonically with twist number. Instead, the tensile strength increased with twist number up to a threshold, above which the tensile strength was observed to decrease. Sample data are presented in Table 4. Without being bound by theory, twists imparted to the chains of polymers in the electrospun sutures may increase the tensile strength by partially distributing a force directed along the suture linear axis into vectors orthogonal to the linear axis of the polymer chain. However, the twists may also impart rotational stress to the polymers. As a result, an excessive twist number may result in the addition of significant rotational stress to the suture, thereby resulting in an overall decrease in the tensile strength.

**Table 4**

| Twist Number | Tensile Strength (MPa) |
|---|---|
| 0 | 43.7 |
| 500 | 52.9 |
| 5000 | 37.2 |

### Example 5: Braided Electrospun Sutures (for reference)

Three single strands of nanofiber material were braided together to form a nanofiber braided suture. Multiple samples were fabricated for statistical testing. The sample single nanofiber strands were prepared as disclosed in Example 3. After each nanofiber strand was fabricated, three of the nanofiber strands were braided together by attaching the ends of the nanofiber material to a fixed point and crossing the strands over each other until the whole length was braided. Table 5 discloses some properties of examples of such sutures.

**Table 5**

| Sample Number | Tensile Strength (MPa) | % Elongation | Diameter (µm) | Break Force (N) |
|---|---|---|---|---|
| 1 | 54.4 | 56 | 266 | 1.06 |
| 2 | 20.0 | 54 | 339 | 0.61 |
| 3 | 12.2 | 45 | 535 | 0.94 |
| 4 | 12.7 | 33 | 337 | 0.38 |
| Ave. | 24.8 | 47 | 369 | 0.75 |
| Stdev. | 20.1 | 10 | 115 | 0.31 |
| St. Error of the Mean | 8.97 | 4.6 | 51.6 | 0.14 |

The braided electrospun suture had an average USP size of less than 2-0.

### Example 6: Micronized Electrospun Textile Fragments

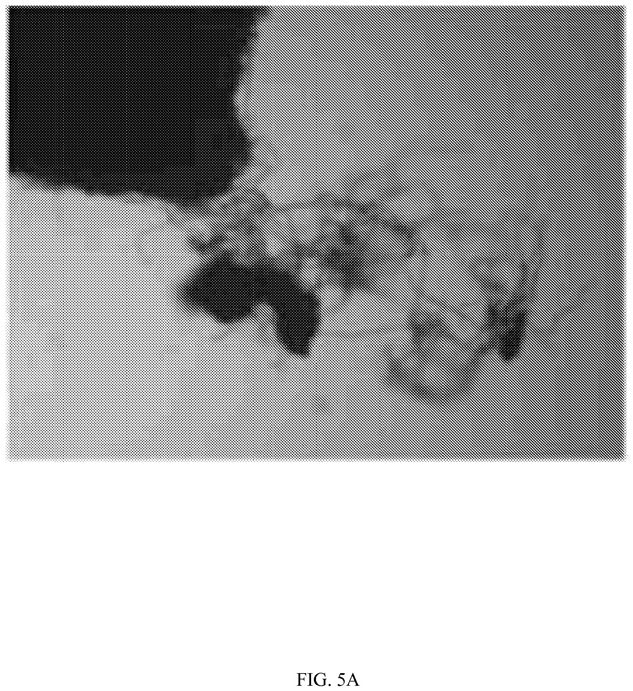

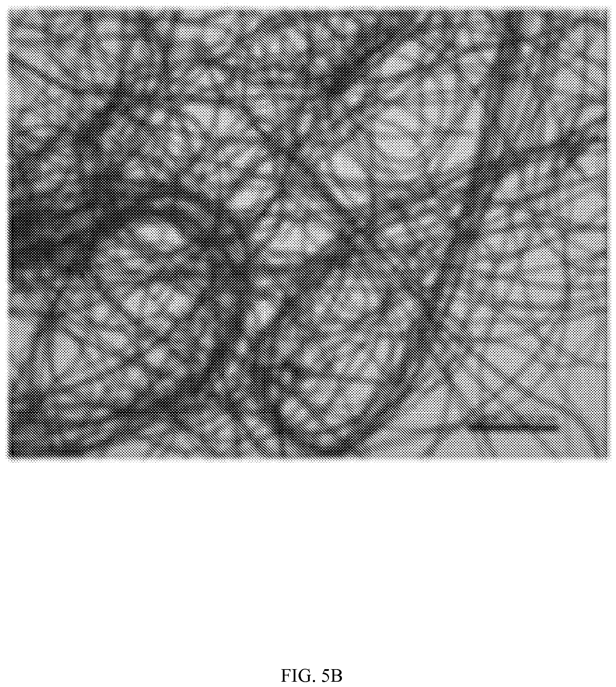

FIGS. 5A and 5B depict images of micronized electrospun textile fragments dispersed in water. The textile fragments were prepared by a standard electrospinning approach and then cryosheared. Briefly, 8 wt% polylactic acid was dissolved in hexafluoro isopropanol and electrospun into a non-woven mat. The mat was then cut into approximately 5 mm x 5 mm pieces and placed in liquid nitrogen. A shear mixer was then placed in the liquid nitrogen at approximately 30,000 RPM for 1 minute to micronize the fibers. FIG. 5A depicts a low power magnification (40x) view, and FIG. 5B depicts a high power magnification (100x) view of the electrospun textile fragments. The micronized electrospun textile fragments depicted in FIGS. 5A and 5B had an average diameter of 500 nm and an average length of about 500 µm.

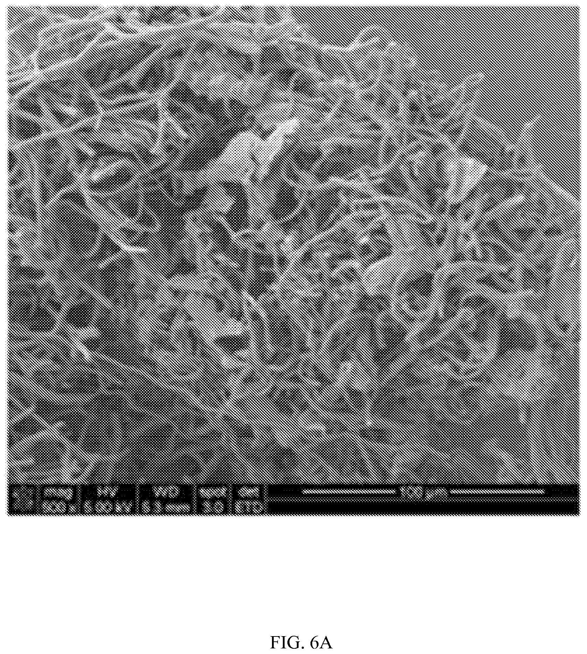

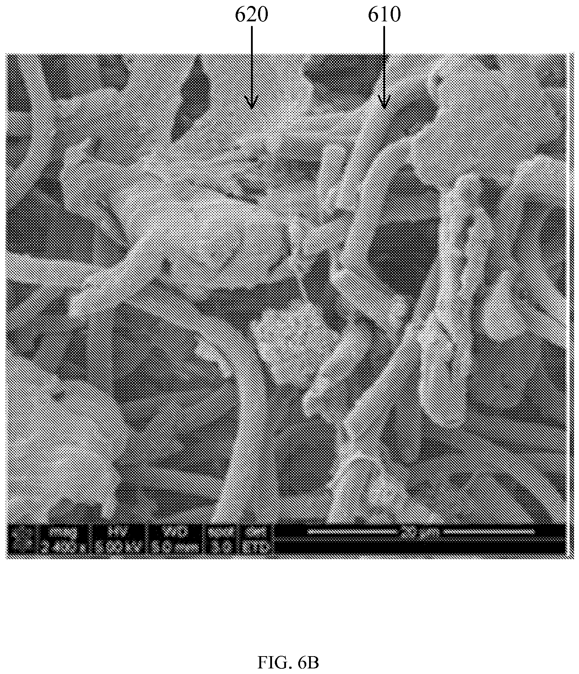

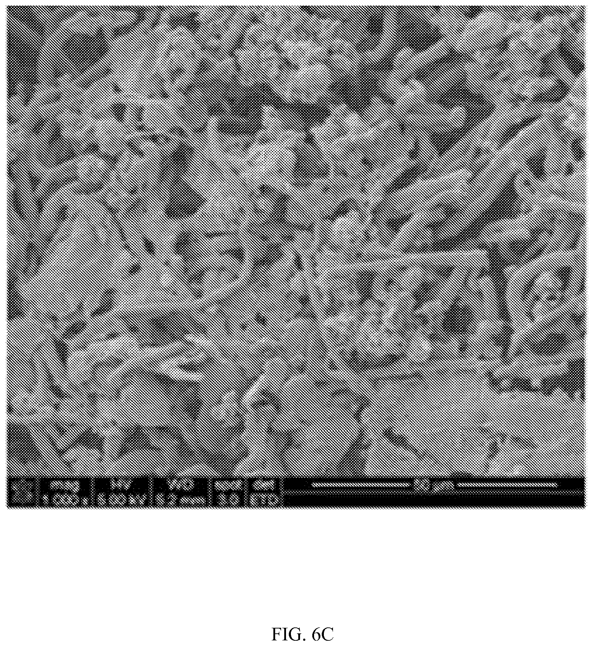

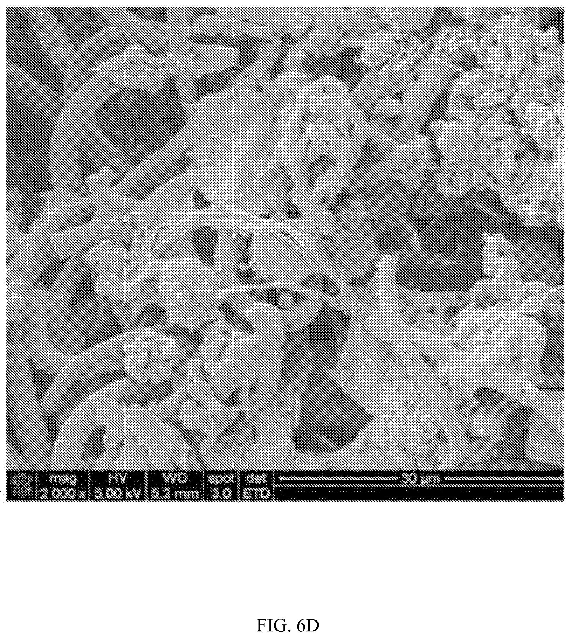

Approximately 1.5 mg of micronized nanofiber fragments of polylactic acid fibers (500nm diameter, 500µm length) was mixed with adipose derived mesenchymal stem cells using the stromal vascular fraction suspended in phosphate buffered saline and maintained at room temperature for up to four hours. FIG. 6A depicts a micrograph of the suspension of micronized nanofiber fragments immediately after the addition of the stem cells. FIG. 6B depicts the same preparation as FIG. 6A after an incubation time of about 5 minutes, and shows a cluster of nanofiber fragments **610**, and a cell embedded in the suspension of micronized nanofiber fragments **620.** FIG. 6C depicts the same preparation after an incubation time of about 15 minutes, and FIG. 6D depicts the same preparation after an incubation time of about 30 minutes. It may be observed that the stem cells quickly attach, proliferate, and produce extracellular matrix on the nanofibers, and appear to totally cover the nanofiber textile fragments in about 2 hours.

### Example 7: "Nanowhisker" Loadings and Syringe Tip Gauges

In an experiment, 1.5 mL vials loaded with electrospun nanofiber fragments and clusters, or "nanowhiskers," as described above, were filled with 1 mL phosphate buffered saline (PBS). Using a 20 cc syringe, the suspension of electrospun nanofiber fragments and clusters was pulled out of the vial and into the syringe. The syringe and empty vial were both inspected for the presence of the electrospun nanofiber fragments and clusters, and the suspension was then injected back into the vial. The full vial and empty syringe were then both inspected for the presence of the electrospun nanofiber fragments and clusters. This procedure was repeated for each loading, using syringe tips with progressively smaller diameters. Syringe tips were flushed with isopropanol, followed by PBS, between each test.

At nanofiber fragment and/or cluster concentrations of 1 mg/mL, 2 mg/mL, 3 mg/mL, 5 mg/mL, 10 mg/mL, and 15 mg/mL, with an at least 18-gauge syringe tip, and at 1 mg/mL, 2 mg/mL, 3 mg/mL, 5 mg/mL, and 10 mg/mL, with an at least 20-gauge syringe tip, the suspension with electrospun nanofiber fragments and clusters completely passed into and out of the syringe tip. No nanofiber material was left on the syringe tip when the suspension was pulled into the syringe, and little or no nanofiber material was left inside the syringe after the suspension had been injected back into the vial.

### Example 8: "Nanowhisker" Implantation in Equine Subjects

The injection of a suspension of electrospun nanofiber fragments and clusters, as demonstrated in FIGS. 6A, 6B, 6C, and 6D, was examined in three equine subjects. The first equine subject was a 23-year-old male quarter horse with a history of chronic bilateral front foot pain that did not respond to conventional treatment. The pain was consistent with caudal foot pain and navicular syndrome. On the day of the procedure, the first equine subject was evaluated for lameness, and scored a 3/5 on the American Association of Equine Practitioners (AAEP) lameness scale.

The second equine subject was a 23-year-old male quarter horse who scored 1/5 on the AAEP lameness scale after an acute soft tissue injury to his left stifle joint. The second equine subject showed minimal response to conventional joint therapies prior to the procedure.

The third equine subject was a 20-year-old female quarter horse with a history of chronic degenerative joint disease in her hind limbs, which showed limited response to conventional therapies. She scored 1/5 on the AAEP lameness scale at the trot, and 4/5 after stifle flexion.

Blood was drawn and adipose tissue was harvested from the rump of each subject. The adipose samples were incubated in a hot water bath, and stem cells were isolated using enzymatic digestion, centrifugation, and a vacuum-powered sieve. Platelet-rich plasma was isolated from the blood samples via centrifugation, and was added to the adipose stem cell suspensions. These suspensions were placed in an LED stem cell activation device, which activated the cells to more quickly begin the repair process upon re-implantation. Stem cell suspensions were then removed from the LED device, and placed in sterile vials with a concentration of 2 mg electrospun nanofiber fragments and clusters per 1 mL cell suspension. These vials were gently agitated to disperse the nanofibers in the stem cell solution, and then left to sit for 15 minutes to allow the autologous stem cells to adhere to the electrospun nanofiber fragments and clusters. The resultant suspensions were drawn into sterile syringes with 20-gauge needle tips for injection into the joints.

The first equine subject received injections in each of his front coffin joints. The second equine subject received an injection in the medial femorotibial joint of his left stifle. The third equine subject received injections in the medial femorotibial joints of both stifles. All three horses were treated with phenylbutazone for 4 days following the procedure. After 30 days, each horse was re-evaluated. The first equine subject improved from a 3/5 score to a 1/5 score on the AAEP lameness scale, the second equine subject scored slightly less than 1/5 with a 50% improvement in flexion, and the third equine subject showed a 25% improvement in stifle flexion. Each subject showed improvements in the lameness and flexion of the affected joints, which surpassed those gained from conventional treatments.

While the present disclosure has been illustrated by the description of exemplary embodiments thereof, and while the embodiments have been described in certain detail, it is not the intention of the Applicants to restrict or in any way limit the scope of the appended claims to such detail. Additional advantages and modifications will readily appear to those skilled in the art. Therefore, the disclosure in its broader aspects is not limited to any of the specific details, representative devices and methods, and/or illustrative examples shown and described.

## Claims

1. A composition comprising:
a plurality of micronized electrospun fiber fragments comprising a polymer, wherein the plurality of electrospun fiber fragments have an average length of 0.9 µm to 1100 µm; and an average diameter of 0.09 µm to 11 µm;
a plurality of electrospun fiber fragment clusters consisting of the polymer, wherein the plurality of electrospun fiber fragment clusters have independently, an average length of 0.9 µm to 1100 µm, an average width of 0.9 µm to 1100 µm, and an average height of 0.9 µm to 1100 µm; and
a carrier medium.

2. The composition of claim 1, wherein the carrier medium is a phosphate buffered saline, a cell culture media, a platelet-rich plasma, a plasma, a lactated Ringer's solution, a gel, a stromal vascular fraction, or any combination thereof.

3. The composition of claim 1 or 2, further comprising a plurality of cells.

4. The composition of claim 3, wherein the plurality of cells comprises differentiated cells, multipotent stem cells, pluripotent stem cells, totipotent stem cells, autologous cells, syngeneic cells, allogeneic cells, or any combination thereof.

5. The composition of claim 1, wherein a weight percent of the plurality of electrospun fiber fragments and the plurality of electrospun fiber fragment clusters to the carrier medium is about 0.001 wt% to about 50 wt%.

6. The composition of any one of claims 1 to 2, wherein the plurality of electrospun fiber fragments further comprise a radiation opaque material, an electrically conductive material, a fluorescent material, a luminescent material, an antibiotic, a growth factor, a vitamin, a cytokine, a steroid, an anti- inflammatory drug, a small molecule, a sugar, a salt, a peptide, a protein, a cell factor, a DNA, an RNA, or any combination thereof.

7. A kit comprising:
a first component comprising;
a plurality of micronized electrospun fiber fragments comprising a polymer, wherein the plurality of electrospun fiber fragments have an average length of 0.9 µm to 1100 µm; and an average diameter of 0.09 µm to 11 µm;
a plurality of electrospun fiber fragment clusters consisting of the polymer, wherein the plurality of electrospun fiber fragment clusters have, independently, an average length of 0.9 µm to 1100 µm, an average width of 0.9 µm to 1100 µm, and an average height of 0.9 µm to 1100 µm; and
a second component comprising a carrier medium.

8. The kit of claim 7, wherein the second component further comprises a plurality of cells.

9. A kit of any one of claims 7 or 8 wherein the carrier medium is a phosphate buffered saline, a cell culture media, a platelet-rich plasma, a plasma, a lactated Ringer's solution, a gel, a stromal vascular fraction, or any combination thereof.

10. A kit of claim 7, wherein the plurality of cells comprises differentiated cells, multipotent stem cells, pluripotent stem cells, totipotent stem cells, autologous cells, syngeneic cells, allogeneic cells, or any combination thereof.

11. A kit according to any one of claims 7 to 10 wherein the plurality of electrospun fiber fragments further comprise a radiation opaque material, an electrically conductive material, a fluorescent material, a luminescent material, an antibiotic, a growth factor, a vitamin, a cytokine, a steroid, an anti- inflammatory drug, a small molecule, a sugar, a salt, a peptide, a protein, a cell factor, a DNA, an RNA, or any combination thereof.

## Patentansprüche

1. Zusammensetzung, umfassend:
eine Vielzahl von mikronisierten elektrogesponnenen Faserfragmenten, die ein Polymer umfassen, wobei die Vielzahl von elektrogesponnenen Faserfragmenten eine durchschnittliche Länge von 0,9 µm bis 1100 µm und einen durchschnittlichen Durchmesser von 0,09 µm bis 11 µm aufweist;
eine Vielzahl von elektrogesponnenen Faserfragmentclustern, die aus dem Polymer bestehen, wobei die Vielzahl von elektrogesponnenen Faserfragmentclustern unabhängig eine durchschnittliche Länge von 0,9 µm bis 1100 µm, eine durchschnittliche Breite von 0,9 µm bis 1100 µm und eine durchschnittliche Höhe von 0,9 µm bis 1100 µm aufweist; und
ein Trägermedium.

2. Zusammensetzung nach Anspruch 1, wobei das Trägermedium eine phosphatgepufferte Salzlösung, ein Zellkulturmedium, ein plättchenreiches Plasma, ein Plasma, eine laktierte Ringer-Lösung, ein Gel, eine stromale Gefäßfraktion oder eine beliebige Kombination davon ist.

3. Zusammensetzung nach Anspruch 1 oder 2, ferner umfassend eine Vielzahl von Zellen.

4. Zusammensetzung nach Anspruch 3, wobei die Vielzahl von Zellen differenzierte Zellen, multipotente Stammzellen, pluripotente Stammzellen, totipotente Stammzellen, autologe Zellen, syngene Zellen, allogene Zellen oder eine beliebige Kombination davon umfasst.

5. Zusammensetzung nach Anspruch 1, wobei ein Gewichtsprozent der Vielzahl von elektrogesponnenen Faserfragmenten und der Vielzahl von elektrogesponnenen Faserfragmentclustern zu dem Trägermedium etwa 0,001 Gew.-% bis etwa 50 Gew.-% beträgt.

6. Zusammensetzung nach einem der Ansprüche 1 bis 2, wobei die Vielzahl von elektrogesponnenen Faserfragmenten ferner ein strahlungsundurchlässiges Material, ein elektrisch leitendes Material, ein fluoreszierendes Material, ein lumineszierendes Material, ein Antibiotikum, einen Wachstumsfaktor, ein Vitamin, ein Zytokin, ein Steroid, ein entzündungshemmendes Arzneimittel, ein kleines Molekül, einen Zucker, ein Salz, ein Peptid, ein Protein, einen Zellfaktor, eine DNA, eine RNA oder eine beliebige Kombination davon umfasst.

7. Kit, umfassend:
eine erste Komponente, umfassend;
eine Vielzahl von mikronisierten elektrogesponnenen Faserfragmenten, die ein Polymer umfassen, wobei
die Vielzahl von elektrogesponnenen Faserfragmenten eine durchschnittliche Länge von 0,9 µm bis 1100 µm und einen durchschnittlichen Durchmesser von 0,09 µm bis 11 µm aufweist;
eine Vielzahl von elektrogesponnenen Faserfragmentclustern, die aus dem Polymer bestehen, wobei die Vielzahl von elektrogesponnenen Faserfragmentclustern unabhängig eine durchschnittliche Länge von 0,9 µm bis 1100 µm, eine durchschnittliche Breite von 0,9 µm bis 1100 µm und eine durchschnittliche Höhe von 0,9 µm bis 1100 µm aufweist; und
eine zweite Komponente, die ein Trägermedium umfasst.

8. Kit nach Anspruch 7, wobei die zweite Komponente ferner eine Vielzahl von Zellen umfasst.

9. Kit nach einem der Ansprüche 7 oder 8, wobei das Trägermedium eine phosphatgepufferte Salzlösung, ein Zellkulturmedium, ein plättchenreiches Plasma, ein Plasma, eine laktierte Ringer-Lösung, ein Gel, eine stromale Gefäßfraktion oder eine beliebige Kombination davon ist.

10. Kit nach Anspruch 7, wobei die Vielzahl von Zellen differenzierte Zellen, multipotente Stammzellen, pluripotente Stammzellen, totipotente Stammzellen, autologe Zellen, syngene Zellen, allogene Zellen oder eine beliebige Kombination davon umfasst.

11. Kit nach einem der Ansprüche 7 bis 10, wobei die Vielzahl von elektrogesponnenen Faserfragmenten ferner ein strahlungsundurchlässiges Material, ein elektrisch leitendes Material, ein fluoreszierendes Material, ein lumineszierendes Material, ein Antibiotikum, einen Wachstumsfaktor, ein Vitamin, ein Zytokin, ein Steroid, ein entzündungshemmendes Arzneimittel, ein kleines Molekül, einen Zucker, ein Salz, ein Peptid, ein Protein, einen Zellfaktor, eine DNA, eine RNA oder eine beliebige Kombination davon umfasst.

## Revendications

1. Composition comprenant :
une pluralité de fragments de fibres électrofilées micronisées comprenant un polymère, dans laquelle la pluralité de fragments de fibres électrofilées ont une longueur moyenne de 0,9 µm à 1 100 µm ; et un diamètre moyen de 0,09 µm à 11 µm ;
une pluralité de groupes de fragments de fibres électrofilées constitués du polymère, dans laquelle la pluralité de groupes de fragments de fibres électrofilées ont indépendamment, une longueur moyenne de 0,9 µm à 1 100 µm, une largeur moyenne de 0,9 µm à 1 100 µm, et une hauteur moyenne de 0,9 µm à 1 100 µm ;
et un milieu de support.

2. Composition selon la revendication 1, dans laquelle le milieu de support est une saumure tamponnée au phosphate, un milieu de culture cellulaire, un plasma riche en plaquettes, un plasma, une solution de Ringer lactée, un gel, une fraction vasculaire stromale, ou toute combinaison de ceux-ci.

3. Composition selon la revendication 1 ou 2, comprenant en outre une pluralité de cellules.

4. Composition selon la revendication 3, dans laquelle la pluralité de cellules comprend des cellules différenciées, des cellules souches multipotentes, des cellules souches pluripotentes, des cellules souches totipotentes, des cellules autologues, des cellules syngéniques, des cellules allogéniques, ou toute combinaison de celles-ci.

5. Composition selon la revendication 1, dans laquelle un pourcentage pondéral de la pluralité de fragments de fibres électrofilées et de la pluralité de groupes de fragments de fibres électrofilées par rapport au milieu de support est d'environ 0,001 % en poids à environ 50 % en poids.

6. Composition selon l'une quelconque des revendications 1 à 2, dans laquelle la pluralité de fragments de fibres électrofilées comprend en outre un matériau radio-opaque, un matériau conducteur électriquement, un matériau fluorescent, un matériau luminescent, un antibiotique, un facteur de croissance, une vitamine, une cytokine, un stéroïde, un médicament anti-inflammatoire, une petite molécule, un sucre, un sel, un peptide, une protéine, un facteur cellulaire, un ADN, un ARN, ou toute combinaison de ceux-ci.

7. Trousse comprenant :
un premier composant comprenant :
une pluralité de fragments de fibres électrofilées micronisées comprenant un polymère, dans laquelle la pluralité de fragments de fibres électrofilées ont une longueur moyenne de 0,9 µm à 1 100 µm ; et un diamètre moyen de 0,09 µm à 11 µm ;
une pluralité de groupes de fragments de fibres électrofilées constitués du polymère, dans laquelle la pluralité de groupes de fragments de fibres électrofilées ont indépendamment, une longueur moyenne de 0,9 µm à 1 100 µm, une largeur moyenne de 0,9 µm à 1 100 µm, et une hauteur moyenne de 0,9 µm à 1 100 µm ;
et un deuxième composant comprenant un milieu de support.

8. Trousse selon la revendication 7, dans laquelle le deuxième composant comprend en outre une pluralité de cellules.

9. Trousse selon l'une quelconque des revendications 7 ou 8 dans laquelle le milieu de support est une saumure tamponnée au phosphate, un milieu de culture cellulaire, un plasma riche en plaquettes, un plasma, une solution de Ringer lactée, un gel, une fraction vasculaire stromale, ou toute combinaison de ceux-ci.

10. Trousse selon la revendication 7, dans laquelle la pluralité de cellules comprend des cellules différenciées, des cellules souches multipotentes, des cellules souches pluripotentes, des cellules souches totipotentes, des cellules autologues, des cellules syngéniques, des cellules allogéniques, ou toute combinaison de celles-ci.

11. Trousse selon l'une quelconque des revendications 7 à 10 dans laquelle la pluralité de fragments de fibres électrofilées comprend en outre un matériau radio-opaque, un matériau conducteur électriquement, un matériau fluorescent, un matériau luminescent, un antibiotique, un facteur de croissance, une vitamine, une cytokine, un stéroïde, un médicament anti-inflammatoire, une petite molécule, un sucre, un sel, un peptide, une protéine, un facteur cellulaire, un ADN, un ARN, ou toute combinaison de ceux-ci.
